(19) Europäisches Patentamt
European Patent Office
Office européen des brevets

(11) Publication number: **0 289 801 B1**

(12) **EUROPEAN PATENT SPECIFICATION**

(45) Date of publication of the patent specification: **07.11.90**

(51) Int. Cl.[5]: **C07D 487/04**, C07D 519/00, A61K 31/395

(21) Application number: **88105608.9**

(22) Date of filing: **08.04.88**

(54) **(1R,5S,6S)-2-(substituted thio)-6-[(R)-1-hydroxyethyl]-1-methyl-carbapenem-3-carboxylic-acid derivatives.**

(30) Priority: **11.04.87 JP 89016/87**
**15.02.88 JP 31033/88**

(43) Date of publication of application:
**09.11.88 Bulletin 88/45**

(45) Publication of the grant of the patent:
**07.11.90 Bulletin 90/45**

(84) Designated Contracting States:
**AT BE CH DE ES FR GB GR IT LI LU NL SE**

(56) References cited:
**EP-A- 0 008 514**
**EP-A- 0 165 384**
**EP-A- 0 170 073**
**US-A- 4 644 061**

**ANGEWANDTE CHEMIE, Int. Ed., vol. 24, 1985; p. 180**
**JOURNAL MED. CHEMISTRY, vol. 26, 1983; p. 259**
**COMPREHENSIVE HETEROCYCLIC CHEM., Chem. of Heterocyclic Comp.: "1,2,4-triazoles" and "Synthesis of fused heterocycles"**

(73) Proprietor: **LEDERLE (JAPAN) LTD., Hattori Bldg., 5th Floor 10-3 Kyobashi 1-chome, Chuo-ku Tokyo(JP)**

(72) Inventor: **Kumagai, Toshi, 3-15-37, Kosenba-cho, Kawagoe-shi Saitama-ken(JP)**
Inventor: **Matsunaga, Hiroshi, 3-6-3-307, Niiza, Niiza-shi Saitama-ken(JP)**
Inventor: **Machida, Yoshisuke, 1-18-23, Kamimuneoka, Shiki-shi Saitama-ken(JP)**
Inventor: **Nagase, Yunosuke, 3-2-7, Nishi-oizumi Nerima-ku, Tokyo(JP)**
Inventor: **Hikida, Muneo, 303-201, Harigaya-nakadohri, Fujimi-shi Saitama-ken(JP)**
Inventor: **Nagao, Yoshimitsu, Gokanosho-shukusha, Uji-shi Kyoto-fu(JP)**

(74) Representative: **Kraus, Walter, Dr. et al, Patentanwälte Kraus, Weisert & Partner Thomas-Wimmer-Ring 15, D-8000 München 22(DE)**

**EP 0 289 801 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**Description**

The present invention relates to a carbapenem antibiotic and, more particularly, to a 1β-methyl-carbapenem derivative having a methyl group introduced at the 1-position of the carbapenem skeleton to an antibacterial composition containing the same as an active ingredient, and to a process of preparing the same.

Heretofore, as various antibacterial substances, there have been proposed many carbapenem antibiotic substances having, as a basic skeleton, carba-2-penem-3-carboxylic acid represented by the following formula (A):

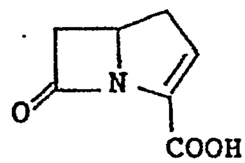

(A)

For example, an initial generation of carbapenem antibiotics is a naturally occurring carbapenem compound such as thienamycin represented by the formula (B):

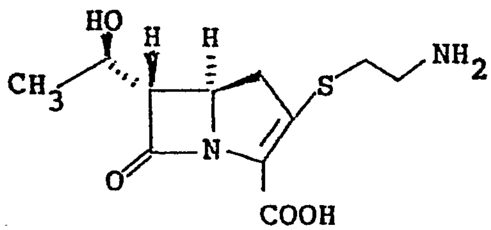

(B)

The thienamycin may be obtained from a fermentation broth of <u>Streptomyces cattleya</u> and has a broad range of antibacterial spectra against Gram-positive and Gram-negative bacteria. It has been expected therefore to be developed as a highly useful compound, but its poor chemical stability has precluded its commercialization.

With the foregoing background, many researchers have attempted to develop a carbapenem compound having antibacterial activities as high as thienamycin and ensuring more chemical stability. As a result, there has been developed imipenem (INN) represented by the following formula (C):

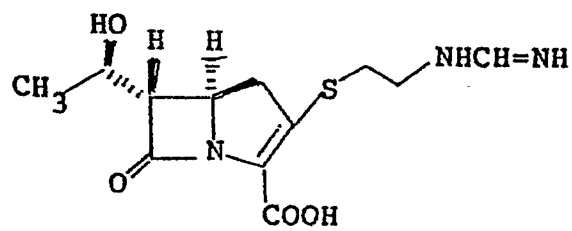

(C)

This compound is a practically available antibacterial agent and may be obtained by converting an amino group as a side chain at the 2-position to a formimidoyl group.

The imipenem of the formula (C) exhibits antibacterial activities higher than those of the thienamycin and ensures some degree of chemical stability; however, it presents the disadvantage that it is decomposed within a short period of time by kidney dehydropeptidase (DHP) in the living body. For this reason, it cannot be administered singly, and must be used in combination with a DHP inhibitor in order to control its decomposition leading to inactivation. Its formulation for clinical administration is a combination with cilastatin (INN) that is a DHP inhibitor.

An antibacterial agent preferred for practical clinical use, however, is one that alone can demonstrate antibacterial activity. Furthermore, the DHP inhibitor to be combined with the antibiotic could exert undesirable actions on tissues of the living body. For these reasons, the combined use should be avoided wherever possible. Thus there has been a growing demand for a carbapenem compound having sufficiently high degrees of both antibacterial activity and resistance to DHP.

Recently, there were proposed some carbapenem compounds of the type that could achieve the above objectives. Such carbapenem compounds are 1-methyl-carbapenem compounds in which a methyl

group is introduced at the 1-position of the carbapenem skeleton. Most recently, another type of carbapenem compounds was proposed which has a heterocycloalkylthio group at the 2-position of the carbapenem skeleton. For example, European Patent Publication No. 170,173 (Japanese Laid-Open Patent Publication No. 83,183/1986) to Merck discloses 1-methylcarbapenem compounds having at the 2-position an alkylated mono- or bi-cyclic quaternary heteroaryl alkylthio substituent, represented by the formula (D):

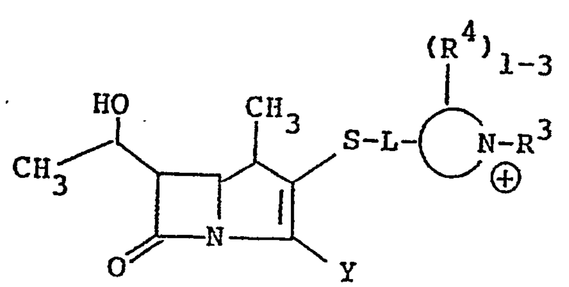

(D)

It is reported that these compounds have superior antibacterial activities as well as a remarkably improved resistance to decomposition by DHP leading to inactivation so that they demonstrate highly useful effects.

It should be noted here that the Japanese Patent Laid-Open Publication No. 83,183/1986 discloses merely a general concept of 1β-methyl-carbapenem compounds and a very limited number of specific working examples. It also discloses in generic terms that they are superior in antibacterial activities; however, it does not specifically describe any antibacterial data whatsoever. Furthermore, the Japanese patent document merely enumerates more than about 470 compounds by their names only; but it contains as slightly less than 10 compounds that have been actually supported by working examples. No specific compounds according to the present invention are disclosed therein and the prior patent application quoted above does not hint anything about such compounds having superior pharmacological characteristics as demonstrated and claimed by the present invention.

Furthermore, U. S. Patent 4,644,061 assigned to Bristol Myers discloses carbapenem compounds with a quaternary heteroalkylthio substituent at the 2-position of the carbapenem skeleton, as represented by the formula (E):

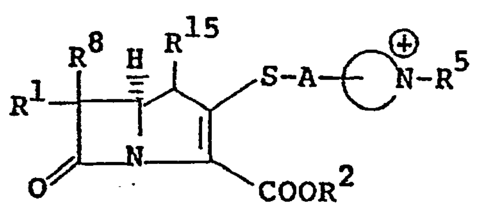

(E)

wherein $R^1$ is a hydroxyethyl group; $R^8$ is a hydrogen atom; and $R^{15}$ is a hydrogen atom or a methyl group.

EP-A 0 165 384 discloses 6-(1-hydroxyethyl)-2(azacycloalkylthio)-carbapenems exhibiting antibiotic properties. In Example 8 of this citation a compound is described which shows good antibacterial activity. It has been said that its stability against DHP is better than the stability of imipenem. However, this compound is decomposed in the in vivo stage and the resulting decomposed substances show renal toxicity. Therefore, in the clinical trial this compound must be used in combination with N-benzoyl-β-alanin as a chemical stabilizer.

The references Cohen, J. Med. Chem. 26 (1983) 259, and Dürckheimer et al., Angew. Chem. Int. Ed. 24 (1985) 180, relate to general articles concerning the recent development of β-lactam antibiotics. Among them, the article of Dürckheimer et al. discloses the compound described in Example 8 of EP-A 0 165 384 and imipenem, among other compounds. As mentioned above, both compounds have the disadvantage that they must be used together with other ingredients.

US-A 4 644 061 discloses carbapenems having a quaternary heteroalkylthio substituent at the 2-position of the carbapenem skeleton, however, the compounds described in this publication have the disadvantages of the carbapenem compounds mentioned above.

In EP-A 0 168 707 (in claim 2 on page 193, line 10) the structural formula of (1R,5S,6S)-2-[6, 7-dihydro5H-pyrazolo[1, 2-a] [1,2,4]triazolium-6-yl)] thio-6-[(R)-1hydroxyethyl]-1-methyl-carbapenem-3-carboxylate is stated.

EP-A 0 168 707 does not disclose any workable Example for the preparation of this compound. It discloses neither physico-chemical data such as NMR, mp, etc., nor biological data to characterize or identify said compound. The compound is listed at the left end of page 140, line 10 in Example 35 as one of 94 compounds which could be produced by using the procedures of Examples 1 to 30 (the same description

is also found at the left end of page 193, line 10 (in claim 2)). It can be assumed that Example 35 is not a real Example but a desk work example. The compounds of the following formulae

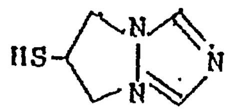 and 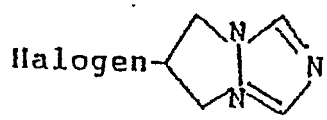

which are the starting materials for the production of (1R,5S,6S)-2-[(6, 7-dihydro-5H-pyrazolo[1, 2-a][1,2,4]triazolium-6-yl)] thio-6-[(R)-1-hydroxyethyl]-1-methyl-carbapenem-3-carboxylate according to the processes disclosed on pages 39 to 42 of Merck's application are still unknown compounds which were at least not known before the filing date of EP-A 0 168 707. All experiments carried out by applicant to synthesize these compounds failed.

For the man skilled in the art it was also not possible to prepare (1R,5S,6S)-2-[(6, 7-dihydro-5H-pyrazolo[1, 2-a] [1,2,4]triazolium-6-yl)] thio-6-[(R)-1-hydroxyethyl]1-methyl-carbapenem-3-carboxylate on the basis of the teaching of the following three references: (i) T. Potts, "Comprehensive Heterocyclic Chemistry", especially page 764, transformation 149 –> 150; the monographs (ii) The Chemistry of Heterocyclic Compounds: "Triazoles 1,2,4"; and (iii) Synthesis of Fused Heterocycles (G.P. Ellis). These references only teach the general method for preparing simple 1,2,4-triazole compounds by reacting unsubstituted hydrazine with amide derivatives (compare reference (i), especially page 763 to 764. It was not possible according to the teaching of these references to prepare (1R,5S,6S)-2-[(6, 7-dihydro-5H-pyrazolo[1, 2-a][1,2,4]triazolium-6-yl]thio-6-[(R)-1-hydroxyethyl]-1-methyl-carbapenem-3-carboxylate.

Since all of these patent applications and references contain only broad generic disclosures and none of them specifically describes the compound of the present invention, they are non-anticipatory of the selective invention disclosed and claimed herein.

The present invention provides carbapenem compounds having high antibacterial activities, a strong action of inhibiting β-lactamase as well as improved resistance to kidney dehydropeptidase. More specifically, the present invention provides the carbapenem compounds substituted by a methyl group at the 1-position in the β-configuration, in which particularly a 4-pyrazolidinyl thio group or a pyrazolotriazolinium-6-yl thio group is introduced at the 2-position.

Specifically, the present invention provides (1R,5S,6S)-2-substituted thio-6-[(R)-1-hydroxyethyl]-1-methyl-carbapenem-3-carboxylic acid derivatives represented by formula (I):

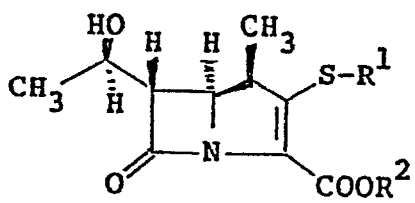

(I)

wherein $R^1$ represents a radical of the formula

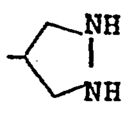 or 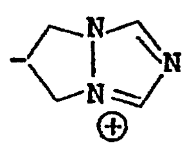

and $R^2$ is a hydrogen atom or an anion charge, or a pharmaceutically acceptable salt thereof.

The carbatenem compounds of the invention, of formula (I) are (1R,5S,6S)-2-[4-pyrazolidinyl]thio-6-[(R)-1-hydroxyethyl]-1-methyl-carbapenem-3-carboxylic acid as formula (I-1):

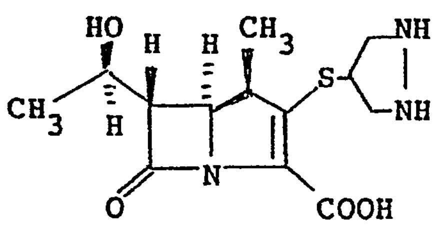

(I-1)

or a pharmaceutically acceptable salt thereof, and (1R,5S,6S)-2-[(6,7-dihydro-5H-pyrazolo[1,2-a][1,2,4]triazolium-6-yl)]thio-6-[(R)-1-hydroxyethyl]-1-methyl-carbapenem-3-carboxylate as formula (I-2):

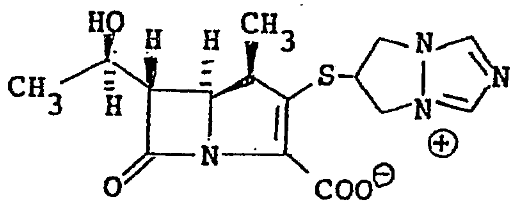

(I-2)

or a pharmaceutically acceptable salt thereof.

Furthermore, the present invention provides a crystalline carbapenem compound of the formula (I-2) or a pharmaceutically acceptable salt thereof.

The carbapenem compounds according to the present invention are novel compounds that are not specifically disclosed in the prior patent publications (for instance, Japanese Patent Laid-Open Publication No. 83,183/1986). In particular, they are remarkably characterized in that the substituent at the 2-position of the carbapenem skeleton is a (5H-pyrazolo[1,2-a][1,2,4]triazolium-6-yl)thio group and in that they have superior antibacterial activities and resistance to DHP.

In accordance with the present invention, the carbapenem compounds represented by formula (I-1) may be prepared basically by a process to be described below.

Briefly stated, the carbapenem of formula (I-1) or pharmacologically acceptable salt thereof may be prepared by reacting a compound represented by formula (II):

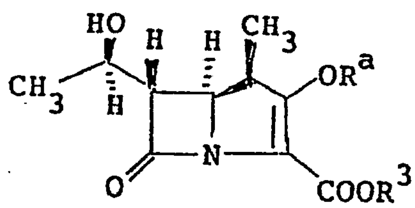

(II)

wherein $R^3$ is a carboxyl protecting group, and $R^a$ is an acyl group, with a mercapto reagent represented by formula (III):

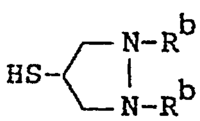

(III)

wherein $R^b$ is an amino protecting group, to give a compound represented by formula (IV):

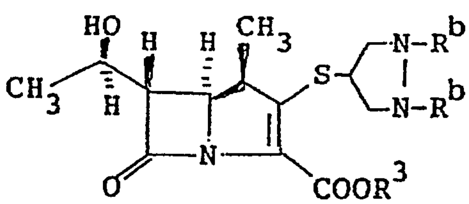

(IV)

wherein $R^3$ and $R^b$ have the same meanings as above, and subjecting the compound of the formula (IV) to removal of the protecting groups $R^3$ and $R^b$ to give the carbapenem compound of the formula (I-1)

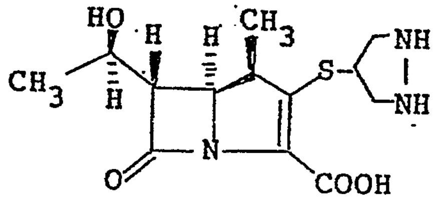

(I-1)

The carbapenem of formula (I-2) or a pharmacologically acceptable salt thereof may be prepared by reacting the above-mentioned compound of formula (I-1) with formimidic acid ester to give the carbapenem compound represented by formula (I-2).

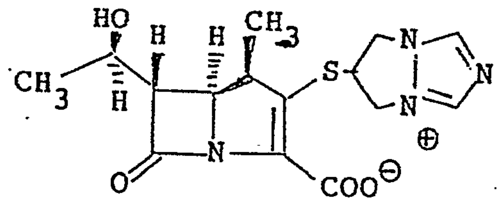

(I-2)

More specifically, the carbapenem compounds represented by formula (I) may be prepared in such a manner as will be described in detail below.

The carbapenem compound represented by formula (II) to be employed as a starting compound in the process described above is known per se and may be prepared in such a manner as disclosed, for example, in U. S. Patent 4,312,871 (Japanese Patent Laid-Open Publication No. 123,985/1981) or, more preferably, in accordance with the spatially selective method as indicated in Reaction Scheme A below and proposed by the present inventors (as disclosed, for example, in Japanese Patent Application No. 315,444/1986).

REACTION SCHEME A

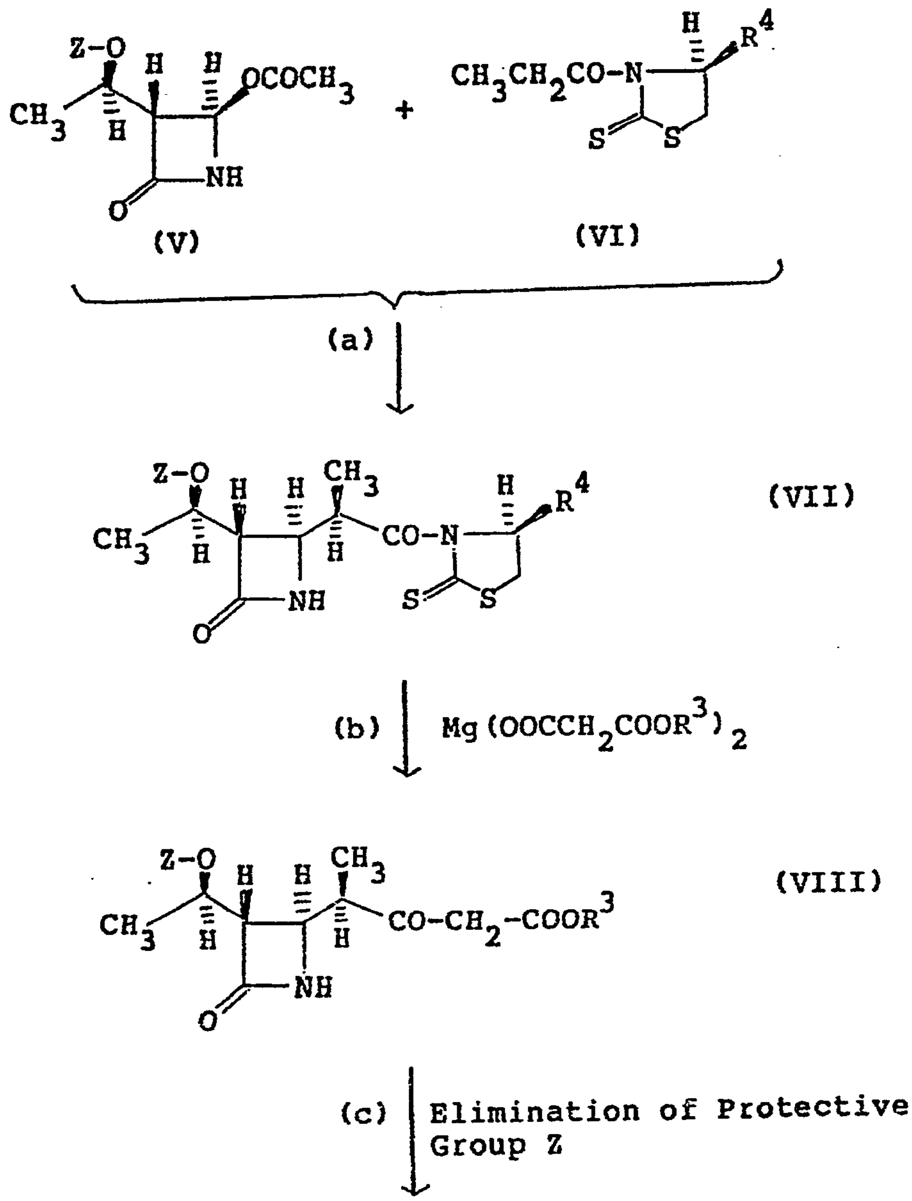


(V) (VI) (VII) (VIII)

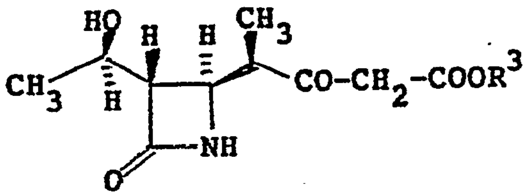

(IX)

(d) Azide Compound/Base

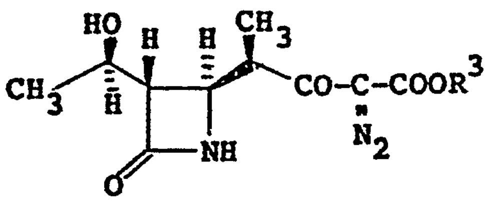

(X)

(e) Cyclization/Metal Catalyst

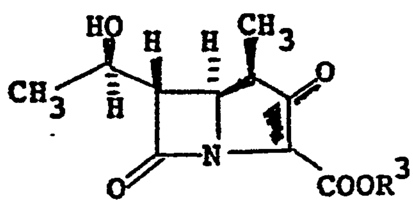

(XI)

(f) Reactive Derivative of $R^aOH$

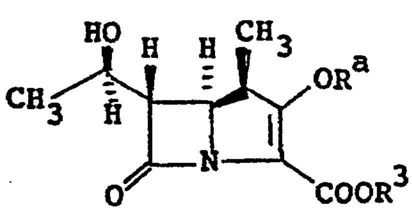

(II)

wherein $R^4$ is a hydrogen atom or a lower alkyl group; Z is tertiary-butyldimethylsilyl group; and $R^3$ and $R^a$ have the same meanings as above.

In the specification of the present application, the term "lower" qualifying a group of a compound means that the group or compound so qualified has from 1 to 7, preferably from 1 to 4, carbon atoms.

The term "lower alkyl" referred to herein stands for a straight-chained or branched-chain hydrocarbon group having preferably from 1 to 6 carbon atoms and may include, for example, methyl, ethyl, n-propyl, isopropyl, n-butyl, isobutyl, sec-butyl, tert.-butyl, n-pentyl, isopentyl, n-hexyl, isohexyl or the like.

The term "carboxyl protecting group" referred to herein stands for any group capable of protecting the carboxyl group of the compound involved without adversely affecting any other substituents and the reactions that follow and may include, for example, an ester residue such as a lower alkyl ester residue

including, for example, methyl ester, ethyl ester, n-propyl ester, isopropyl ester, n-, iso-, sec- or tert.-butyl ester, n-hexyl ester or the like; an aralkyl ester residue including, for example, benzyl ester, p-nitrobenzyl ester, o-nitrobenzyl ester, p-methoxybenzyl ester or the like; and a lower aliphatic acyloxymethyl ester residue including, for example, acetoxymethyl ester, propionyloxymethyl ester, n- or iso-butyryloxymethyl ester, pivaloyloxymethyl ester or the like.

The term "acyl group" referred to herein stands for, in a narrower sense, a moiety obtainable by removing the hydroxyl group from the carboxyl group of an organic carboxylic acid as well as, in a broader sense, any acyl group derived from an organic sulfonic acid or an organic phosphoric acid. Such an acyl group may include, for example, a lower alkanoyl group such as acetyl, propionyl, butyryl or the like, a (halo)lower alkyl sulfonyl group such as methanesyulfonyl, trifluoromethanesulfonyl or the like; a substituted or unsubstituted arylsulfonyl group such as benzenesulfonyl, p-nitrobenzenesulfonyl, p-bromobenzenesulfonyl, toluenesulfonyl, 2,4,6-triisopropylbenzenesulfonyl or the like; and diphenylphosphoryl.

The term "amino protecting group" referred to herein stands for groups usually employed in peptide chemistry, for example, phthaloyl, benzyloxycarbonyl, tert.-butoxycarbonyl, p-nitrobenzyloxycarbonyl or the like.

When $R^2$ in formula (I) represents an anion charge, $-COOR^2$ bonded to the 3-position of the carbapenem skeleton is $-COO^{\ominus}$, and $-COO^{\ominus}$ forms an intramolecular salt with

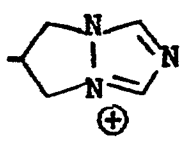 represented by $R^1$.

represented by $R^1$.

Each of the steps of the Reaction Scheme A above for preparing the compounds represented by formula (II) in a highly spatial selectivity will be described below more in detail.

The step (a) involves the reaction of the N-propionyl-1,3-thiazoline-2-thione derivative of formula (VI) with a tin(II) triflate in the presence of a base to give an enolate and then the reaction of the resulting enolate with the compound of formula (V) to give the azetidin-2-one derivative of formula (VII).

The enolization reaction of the N-propionyl-1,3-thiazoline-2-thione derivative of formula (VI) with the tin(II) triflate may be carried out usually in a solvent inert in the reaction, such as an ether, i.e., diethyl ether, tetrahydrofuran or the like; a hydrocarbon, i.e., toluene, cyclohexane or the like; a halogenated hydrocarbon, i.e., dichloromethane, chloroform or the like. Preferably tetrahydrofuran can be used.

The reaction temperature is not limited to a particular range of temperatures and may vary in a wide range with starting materials to be used or the like. Usually the reaction temperature may be in a range of relatively low temperatures as low as from approximately -100°C to about room temperature, preferably from approximately from -78°C to approximately 0°C.

The quantity of the tin(II) triflate with respect to the compound of formula (VI) is not critical and may range usually from approximately 1 mole to approximately 2 moles, preferably from 1 to 1.5 moles, per mole of the compound of formula (VI).

The enolization reaction above is carried out usually in the presence of the base including, for example, a tertiary amine such as triethylamine, diisopropylethyl amine, 1,4-diazabicyclo[2.2.2]octane, N-methylmorpholine, N-ethylpiperidine, pyridine or the like. N-Ethylpiperidine is employed advantageously. The base may be used at a rate ranging generally from approximately 1.0 to approximately 3 molar equivalents, preferably from 1.0 to 2 molar equivalents, per mole of the compound of formula (VI).

The enolization reaction as described above may be completed generally in approximately 5 minutes to approximately 4 hours, thus leading to the formation of the enolate.

After completion of the enolization reaction, the resulting enolate may be used as it is for further reaction with the compound of formula (V).

The resulting enolate is then subjected to the alkylation reaction with the compound of formula (V). The alkylation reaction may be conducted at temperatures in the range generally from approximately -100°C to about room temperature, preferably from approximately -78°C to approximately 10°C. The quantity of the compound of formula (V) is not critical and may vary conveniently in a range generally from approximately 0.5 mole to approximately 5 moles, preferably from 0.5 to 2 moles, per mole of the compound of formula (VI) used for the enolization.

The alkylation reaction may be carried out under such conditions as described above generally for approximately 5 minutes to approximately 5 hours, preferably for 5 minutes to approximately 2 hours.

The enolization and alkylation reactions may be carried out preferably in an inert atmosphere such as an atmosphere of nitrogen gas or argon gas.

The reaction product obtained by the above reaction is then treated with water. For instance, after completion of the reaction, a phosphate buffer with approximately pH 7 is added, and a mixture is then stirred to be followed by filtration of undissolved materials. After filtration, the compound of the formula

(VII) is separated and purified in conventional manner, such as by means of extraction, recrystallization and chromatography.

The step (b) is a step by which the compound of formula (VIII) may be prepared by reacting the azetidin-2-one derivative of formula (VII) obtained by the step (a) above with a magnesium malonate represented by the general formula: $R^3OOCCH_2CO_2)_2Mg$ in the presence of imidazole.

The reaction is carried out preferably in an inert organic solvent such as an ether solvent, i.e., ether, tetrahydrofuran or dioxane; a hydrocarbon solvent, i.e., toluene, xylene or cyclohexane; a halogenated hydrocarbon solvent, i.e., dichloromethane or chloroform; and acetonitrile. Particularly acetonitrile may be employed conveniently.

The reaction temperature is not limited strictly to a particular range and may vary in a wide range with starting materials to be used or the like. They may range generally from approximately 0°C to approximately 100°C, preferably around room temperature.

The quantity of the magnesium malonate with respect to the compound of formula (VII) may be about an equimolar amount, and the reaction may be completed in approximately 50 hours, preferably in approximately 20 hours.

The magnesium malonate to be used may include, for example, p-nitrobenzylmagnesium malonate, benzylmagnesium malonate, methylmagnesium malonate and so on. Among them, p-nitrobenzylmagnesium malonate is preferably used.

The step (c) is a step to eliminate a hydroxyl protecting group Z from the compound of formula (VIII) obtained by the step (b) above. The tertiary-butyl-dimethylsilyl group as the hydroxyl protecting group Z may be eliminated by subjecting the compound of formula (VIII) to acidic hydrolysis in a solvent such as methanol, ethanol, tetrahydrofuran, dioxane or the like in the presence of an acid such as a mineral acid, i.e., hydrochloric acid, sulfuric acid or an organic acid, i.e., acetic acid at temperatures ranging from 0°C to 100°C for reaction periods ranging from 0.5 to 18 hours.

The above step may yield the compound represented by formula (IX) in a quantitative amount.

The step (d) is a step by which the diazo compound of formula (X) may be prepared by treating the compound of formula (IX) obtainable by the above step (c) with an azide compound in the presence of a base in such an inert organic solvent as have been enumerated for the step (d) above.

The azide compound to be used in the step (d) may include, for example, p-carboxylbenzenesulfonyl azide, toluenesulfonyl azide, methanesulfonyl azide, dodecylbenzenesulfonyl azide or the like. The base to be used therein may include, for example, triethylamine, pyridine, diethylamine or the like.

The reaction may be carried out, for instance, by adding p-toluenesulfonyl azide to acetonitrile preferably in the presence of triethylamine at 0°C to 100°C, preferably at room temperature for 1 to 50 hours. This reaction produces the diazo compound represented by formula (X) in a high yield.

The step (e) is a step by which the diazo compound of the formula (X) obtainable by the step (d) above may be cyclized to give the compound of formula (XI). This step may be carried out preferably in an inert solvent such as benzene, toluene, tetrahydrofuran, cyclohexane, ethyl acetate, dichloromethane or the like, preferably in toluene, at temperatures ranging from 25°C to 110°C for 1 to 5 hours in the presence of a metal catalyst such as a metal carboxylate compound including, for example, bis(acetylacetonate)Cu(II), $CuSO_4$, copper powder, $Rh_2(OCOCH_3)_4$, rhodium octanoate, $Pb(OCOCH_3)_4$ or the like. As an alternative procedure, the above cyclization step may be carried out by subjecting the compound of formula (X) to irradiation from a light source through a Pyrex filter (its wavelength being larger than 300 nm) in a solvent such as benzene, diethyl ether or the like at 0°C to 250°C for 0.5 to 2 hours.

The step (f) produces the compound of formula (II) by reacting the compound of formula (XI) obtainable by the step (e) with a reactive derivative of an acid represented by the formula: $R^aOH$. The reactive acid derivative may include, for example, an acid anhydride such as acetic acid anhydride, methanesulfonic acid anhydride, p-toluenesulfonic acid and anhydride, p-nitrobenzenesulfonic acid anhydride, 2,4,6-triisopropylbenzenesulfonic acid anhydride, trifluoromethanesulfonic acid anhydride or the like or an acid halide such as an acid chloride, i.e., acetyl chloride, propionyl chloride, diphenylphosphoric chloride, toluenesulfonyl chloride, p-bromobenzenesulfonyl chloride or the like. Diphenylphosphoric chloride ($R^a$ = diphenylphosphoryl group) is particularly preferred.

The reaction of the compound of formula (XI) with the reactive acid derivative may be carried out, for example, in a manner similar to a conventional acylation reaction in an inert solvent such as methylene chloride, acetonitrile, dimethylformamide or the like, conveniently in the presence of a base such as diisopropylethyl amine, triethylamine, 4-dimethylaminopyridine or the like at temperatures ranging from -20°C to 40°C for approximately 30 minutes to approximately 24 hours.

The reaction consisting of a series of the steps as have been described above provides the compound represented by formula (II) with a highly spatial selectivity and with such a spatial arrangement that the methyl group at the 1-position of the carbapenem skeleton is arranged in the R configuration, the substituent at the 5-position thereof is in the R configuration, and the substituent and the hydroxymethyl group each at the 6-position thereof are in the S and R configurations, respectively.

The compound represented by formula (II) is then reacted with a mercapto reagent represented by formula (III) to give the compound represented by formula (IV).

The reaction of the compound of formula (II) with the mercapto reagent of formula (III) may be carried out, for instance, by reacting the compound of formula (II) with the mercapto reagent of formula (III) in an

excess amount ranging from about an equimolar amount to approximately 1.5 molar amount in an appropriate solvent such as tetrahydrofuran, dichloromethane, dioxane, dimethylformamide, dimethylsulfoxide; acetonitrile, hexamethylene phosphoramide or the like, preferably in the presence of a base such as sodium hydrogen carbonate, potassium carbonate, triethylamine, diisopropylethyl amine or the like at a temperature range from approximately -40°C to approximately 25°C for approximately 30 minutes to approximately 24 hours.

The reaction described above provides the carbapenem compound represented by formula (IV) in which the carboxyl group at the 3-position thereof is protected by the carboxyl protecting group $R^3$ and the substituent at 2-position thereof is protected by the amino protecting group $R^b$. The removal of the protecting groups $R^3$ and $R^b$ may be made by a reaction known per se for removing a protective group, such as solvolysis or hydrogenolysis. In a typical reaction, the compound represented by formula (IV) may be treated, for instance, in a mixture of solvents such as tetrahydrofuran-water, tetrahydrofuran-ethanol-water, dioxane-water, dioxane-ethanol-water, n-butanol-water or the like containing morpholinopropane sulfonic acid-sodium hydroxide buffer solution (pH 7), a phosphate buffer solution (pH 7), dipotassium phosphate, sodium bicarbonate or the like, using hydrogen under 1 to 4 atmospheric pressures, in the presence of a catalyst for hydrogenation such as platinum oxide, palladium-activated carbon or palladium hydroxide-activated carbon at temperatures ranging from approximately 0°C to approximately 50°C for approximately 0.25 to approximately 4 hours.

As a result, (1R,5S,6S)-2-[4-pyrazolidinyl]thio-6-[(R)-1-hydroxyethyl]-1-methyl-carbapenem-3-carboxylic acid represented by formula (I-1), one of the desired compounds of the invention, is produced.

By reacting the resulting compound of formula (I-1) with a formimidic acid ester derivative such as ethyl formimidate hydrochloride, methyl formimidate hydrochloride or benzyl formimidate hydrochloride under weakly basis conditions (for example, in a reaction medium adjusted to a pH of about 8.5 with a phosphate buffer having a pH of 7.0 and a 1N aqueous solution of sodium hydroxide), (1R,5S,6S)-2-[(6,7-dihydro-5H-pyrazolo[1,2-a][1,2,4]triazolium-6-yl)]thio-6-[(R)-1-hydroxyethyl]-1-methyl-carbapenem-3-carboxylate of formula (I-2), another desired compound of the invention.

One presumed course of reaction in the formation of the compound of formula (I-2) by the reaction of the compound of formula (I-1) with the formimidic acid ester derivative is that by the reaction of the compound (I-1) with the formimidic acid ester derivative, (1R,5S,6S)-6-[(R)-1-hydroxyethyl]-1-methyl-2-[(1,2-diiminomethyl)-4-pyrazolidinyl]thiocarbapenem-3-carboxylic acid of the following formula

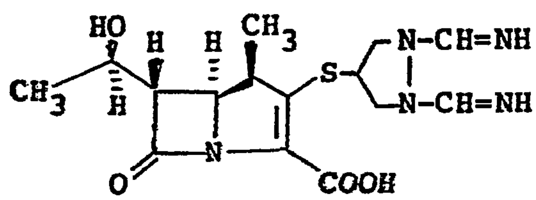

is formed as an intermediate, and this intermediate undergoes cyclization reaction to form the compound of formula (I-2).

In the above reaction, the mercapto reagent of formula (III) is a novel compound not described in the prior literature. It can be obtained, for example, in accordance with the following Reaction Scheme B.

## REACTION SCHEME B

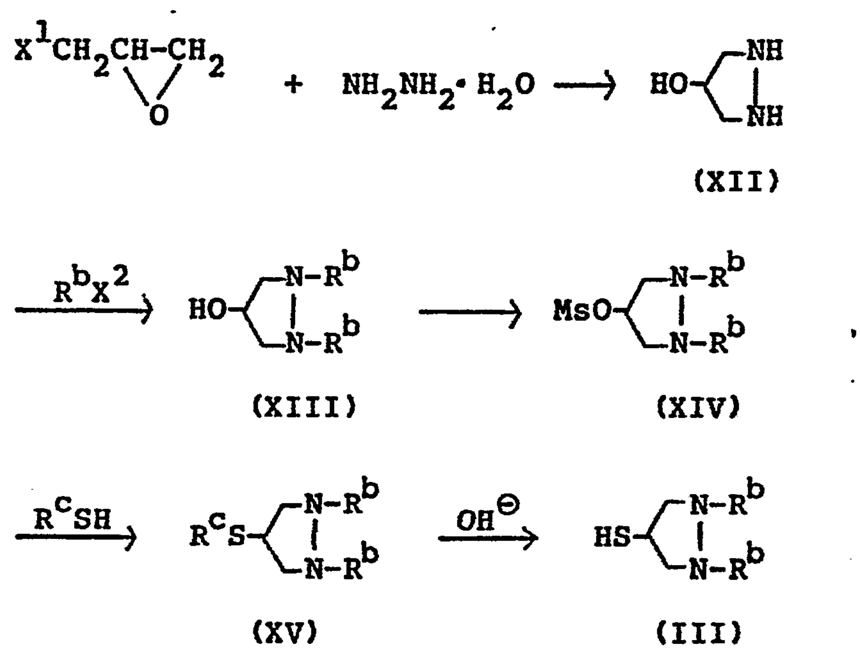


(XII) (XIII) (XIV) (XV) (III)

wherein $R^b$ has the same meaning as above, $X^1$ and $X^2$ are halogen atoms such as a chlorine atom; Ms is methanesulfonyl groups; and $R^c$ is a lower acyl group such as acetyl, propionyl, butyryl group.

4-Hydroxypyrazoline of formula (XII) prepared by the reaction between hydrazine hydrate and epihalohydrin is treated with the acylation reagent of the formula $R^bX^2$ to give the compound of formula (XIII). Then, the resulting compound of formula (XIII) is converted to the compound of formula (XIV) by methanesulfonylation, and the compound of formula (XIV) is reacted with the compound of the formula $R^cSH$ such as thiolacetic acid to obtain the compound of formula (XV). Finally, the resulting compound of formula (XV) is converted to the objective mercapto reagent of formula (III) by reacting with alkali metal alkoxide such as sodium methoxide, sodium ethoxide or the like.

The compound of the present invention represented by formula (I-1) may be converted to a pharmaceutically acceptable salt thereof in a usually manner. Such a salt may be, for example, an alkali metal salt such as sodium, potassium salt thereof; an amino acid salt such as arginine, ornithine, lysine salt thereof; and an ammonium salt such as diethanolammonium, triethanolammonium salt thereof, but the sodium or potassium salt thereof may be more preferable.

In actual application, the (1R,5S,6S)-2-[(6,7-dihydro-5H-pyrazolo[1,2-a][1,2,4]triazolium-6-yl)]thio-6-[(R)]1]hydroxyethyl]-1-methyl-carbapenem-3-carboxylate of formula (I-2) may be converted to a preferred crystalline compound. This crystallization may be carried out by treating the compound of formula (I-2) preferably in water or in an aqueous medium such as water-alcohol, and the resulting crystals may be in the form of an anhydrous compound or a hydrate.

The desired compounds of formula (I) in accord ance with the present invention are novel compounds that are not disclosed specifically in the above-mentioned publication and that are extremely stable against dehydropeptidase (DHP) known as a kidney enzyme and have superior antibacterial activities. The remarkably high antibacterial activities and stability against the kidney DHP of the compounds of formula (I) according to the present invention have been determined by biological tests described below.

I. Antibacterial Tests

Test Procedures:

The antibacterial activities were tested by an agar plate dilution method in accordance with the standard method of The Japanese Chemotherapy Society [Chemotherapy, Vol. 29, 76-79 (1981)].

A Mueller-Hinton (MH) agar liquid medium of a test microorganism was cultured overnight at 37°C and the resultant culture medium was diluted with a buffered saline gelatin (BSG) solution to contain approximately $10^6$ cells of the test microorganisms per milliliter, and then the diluted solution was inoculated with a microplanter at the rate of approximately 5 microliters on a MH agar medium containing a test compound. This medium was then incubated at 37°C for 18 hours. The minimum inhibitory concentration (MIC) is determined as a minimum concentration in which no test microorganism could grow. It is noted here that the test organisms used were all standard strains.

Results:

Table 1 shows the test results. The test compound used therein was the compound (15) obtained in Example 6. As control compounds were used ones clinically employed widely, viz., cefazolin (CEZ) as a cephalosporin compound, and imipenem as a carbapenem compound.

Table 1

MINIMUM INHIBITORY CONCENTRATIONS (MIC)

| Test Organisms | MIC (μg/ml) Test Compounds CEZ | Imipenem | Compd.(15) |
|---|---|---|---|
| Staphylococcus aureus FDA 209P JC-1 | 0.2 | 0.025 | 0.05 |
| Staphylococcus aureus Terajima | 0.05 | <0.006 | 0.025 |
| Staphylococcus aureus MS352 | 0.1 | 0.013 | 0.1 |
| Streptococcus pyogenes Cook | 0.1 | <0.006 | 0.025 |
| Micrococcus luteus ATCC9341 | 0.39 | 0.025 | 0.1 |
| Basillus subtilis ATCC6633 | 0.1 | 0.025 | 0.1 |
| Escherichia coli NIHJ JC-2 | 0.78 | 0.1 | 0.05 |
| Escherichia coli K12 C600 | 0.78 | 3.13 | 0.2 |
| Enterobacter aerougenes ATCC13048 | >100 | 3.13 | 0.39 |
| Enterobacter cloacae 963 | >100 | 0.2 | 0.1 |
| Klebsiella pneumoniae PCI-602 | 0.78 | 0.39 | 0.05 |
| Salmonella typhimurium IID971 | 0.78 | 0.39 | 0.1 |
| Salmonella typhi 901 | 0.78 | 0.1 | 0.025 |
| Salmonella paratyphi 1015 | 1.56 | 1.56 | 0.39 |
| Salmonella schottmuelleri 8006 | 0.78 | 0.78 | 0.2 |
| Salmonella enteritidis G14 | 0.78 | 0.78 | 0.2 |
| Serratia marcescens IAM1184 | >100 | 0.39 | 0.2 |
| Morganella morganii IFO3848 | 25 | 0.39 | 0.1 |
| Proteus mirabilis IFO3849 | 6.25 | 6.25 | 0.39 |
| Proteus vulgaris OX-19 | 6.25 | 0.78 | 0.025 |
| Proteus vulgaris HX-19 | 8.25 | 0.78 | 0.1 |
| Providencia rettgeri IFO 3850 | 12.5 | 0.78 | 0.2 |
| Pseudomonas aeruginosa IFO 3445 | >100 | 0.78 | 0.78 |
| Pseudomanas aeruginosa NCTC 19490 | >100 | 0.78 | 0.78 |

The foregoing results clearly demonstrate that the carbapenem compounds according to the present invention have superior antibacterial activities.

II. Antibacterial Activities against Clinically Isolated β-Lactamase (Cephalosporinase) Producing Strains

Test Procedures:

The antibacterial activities agaisnt clinically isolated β-lactamase producing strains were tested by the agar plate dilution method in accordance with the standard method of The Japanese Chemotherapy Society [Chemotherapy, Vol. 29, 76-79 (1981)]. A solution of a cephalosporinase producing strain stored by Episome Research Institute, prepared by incubating the strain in a sensitivity test broth (STB; product of Nissui K. K.) for 18 hours, was diluted with a fresh STB solution so that the solution contained $10^6$ cells per milliliter. The diluted solution was then inoculated as spots in a microplanter on a sensitivity disk agar-N (SDA; product of Nissui K. K.) containing a test compound. The disk agar was then incubated for 18 to 20 hours. The minimum inhibitory concentration was determined as a minimum concentration in which the test microorganism no longer grew after a 18 to 20-hour incubation.

Results:

Table 2 shows the test results. The test compound used therein was the compound (15) obtained in Example 6. As control compounds, there were used ceftazidime (CAZ) as a cephalosporin compound, and imipenem as a carbapenem compound, both being recognized as having remarkably high antibacterial activities against the test strains and being widely employed clinically.

Table 2

MINIMUM INHIBITORY CONCENTRATIONS (MIC)

| Test Organisms | MIC (μg/ml) Test Compounds CAZ | Imipenem | Compd.(15) |
|---|---|---|---|
| Providencia rettgeri GN 5284 | 0.2 | 0.39 | 0.2 |
| Providencia rettgeri GN 4430 | 0.2 | 0.39 | 0.2 |
| Providencia rettgeri GN 4762 | 0.78 | 0.39 | 0.2 |
| Escherichia coli GN 5482 | 0.78 | 0.1 | 0.025 |
| Escherichia coli No. 1501 | 0.2 | 0.1 | 0.025 |
| Escherichia coli No. 96 | 0.78 | 0.1 | 0.025 |
| Enterobacter cloacae GN 7471 | 3.13 | 0.2 | 0.1 |
| Enterobacter cloacae GN 7467 | 3.13 | 0.78 | 1.56 |
| Enterobacter cloacae GN 5797 | 0.39 | 0.39 | 0.1 |
| Proteus morganii GN 5407 | 0.39 | 3.13 | 1.56 |
| Proteus morganii GN 5307 | 0.2 | 0.78 | 0.39 |
| Proteus morganii GN 5375 | 0.2 | 3.13 | 1.56 |
| Proteus vulgaris GN 76 | 0.1 | 3.13 | 1.56 |
| Proteus vulgaris GN 7919 | 3.13 | 0.39 | 1.56 |
| Proteus vulgaris GN 4413 | 0.2 | 6.25 | 1.56 |
| Pseudomonas aeruginosa GN 918 | 6.25 | 0.78 | 1.56 |
| Pseudomonas aeruginosa GN 10362 | 3.13 | 0.78 | 1.56 |
| Pseudomonas aeruginosa GN 10367 | 3.13 | 1.56 | 1.56 |
| Serratia marcescens GN 10857 | 0.78 | 3.13 | 1.56 |
| Serratia marcescens L-65 | 0.2 | 0.39 | 0.2 |
| Serratia marcescens L-82 | 0.39 | 0.2 | 0.1 |
| Citrobactor freundii GN 346 | 25 | 0.39 | 0.78 |
| Citrobactor freundii GN 7391 | >100 | 0.78 | 0.78 |
| Pseudomonas cepacia GN 11164 | 0.78 | 3.13 | 0.39 |
| Klebsiella oxytoca GN 10650 | 0.2 | 0.2 | 0.1 |

The above results show that the carbapenem compounds according to the present invention had the antibacterial activities against P. aeruginosa and P. cepacia belonging to Pseudomonadaceae as high as imipenem and particularly higher than CAZ having anti-Pseudomonas activities.

It has been further found that the carbapenem compounds of the invention had activities against enteric bacteria excluding the genus Proteus as high as imipenem and superior to CAZ.

III. Sensitivity Tests against Clinical Isolates

1. P. aeruginosa resistant strains

(1) Strains of Test Organisms:

Fifty-four strains of P. aeruginosa demonstrating resistance against the following agents in the concentrations indicated in the following parentheses were employed for sensitivity tests against clinical isolates.
Ceftazidime (CAZ) (25 to 100 μg/ml)21 strains
Cefsulodine (CFS) (25 to >100 μg/ml)23 strains
Piperacilin (PIPC) (25 to >100 μg/ml)15 strains
Gentamycin (GM) (25 to >100 μg/ml)21 strains
Amikacin (AMK) (25 to >100 μg/ml)26 strains
Ofloxacin (OFLX) (25 to >100 μg/ml)4 strains

(2) Test Procedures:

The test procedures were based on the agar plate dilution method in accordance with the standard method of The Japanese Chemotherapy Society. The minimum inhibitory concentration (MIC) was determined in substantially the same manner as the test procedures II above using the 54 strains of P. aeruginosa having an anti-Pseudomonas resistance.

(3) Results:

The compound (15) obtained in Example 6 was found to demonstrate antibacterial activities to inhibit the growth of approximately 98% of the test microorganisms in a concentration of 6.25 μg/ml and all the test microorganisms in a concentration of 12.5 μg/ml.
The imipenem, on the other hand, was found to inhibit the growth of approximately 98% of the test microorganisms in a concentration of 6.25 μg/ml and all the test microorganism in a concentration of 12.5 μg/ml.

2. C. freundii resistant strains

(1) Strains of the Test Organisms:

Twenty-seven strains of C. freundii demonstrating a resistance against the following agent as same manner described above in Test 1.
Cefixime (CFIX) (50 to >100 μg/ml)
Cefotaxime (CTX) (50 to >100 μg/ml)

(2) Test Procedures:

The tests were carried out in the same manner as described above in Test 1.

(3) Results:

The compound (15) obtained in Example 6 was found to demonstrate antibacterial activities to inhibit the growth of approximately 98% of the test microorganisms in a concentration of 0.78 μg/ml and all the test microorganisms in a concentration of 1.56 μg/ml.
The imipenem, on the other hand, was found to inhibit the growth of approximately 90% of the test microorganisms in a concentration of 0.78 μg/ml and all the test microorganisms in a concentration of 1.56 μg/ml.
The above results clearly demonstrate that the compounds according to the present invention have superior antibacterial activities to imipenem.

IV. Stability Test against Kidney Dehydropeptidase:

1. Materials:

(1) Swine Kidney Dehydropeptidase-I (DHP-I):

The swine kidney (8 kg) was homogenized and the enzyme protein was allowed to precipitate. After a connective lipid was removed with acetone, the resultant material was made soluble by treatment with butanol and purified by the ammonium sulfate fraction method, thereby producing DHP-I enzyme from a 75% ammonium sulfate fraction.

The DHP-I enzyme was then adjusted to give an enzyme concentration of 25 mg/10 ml (phosphate buffer, pH 7.1), and divided into 1 ml portions. The portions were frozen and stored at -40°C or less until use.

(2) Test Compound:

Compound (15) obtained in Example 6 below was used as a test compound.

The test compound was adjusted in situ to give a concentration of 117 µM with a 60 mM sodium phosphate buffer solution (pH =7.1).

Glycyl dehydrophenylalanine (Gl-dh-Ph) and imipenem were employed as control compounds, and they were adjusted in situ each to give a concentration of 117 µM with the same sodium phosphate buffer solution.

2. Method:

(1) Measurement for Hydrolysis Activity against DHP-I Enzyme Substrate by Late Assay:

To 1.2 ml of 50 mM sodium phosphate buffer solution (substrate) containing 117 µM of each of Gl-dh-Ph and imipenem as the control compounds was added 0.2 ml of the DHP-I enzyme solution (25 mg/10 ml) prepared above in the final substrate concentration of 100 µM. The solution was then incubated at 37°C for 10 minutes. The initial velocity of hydrolysis of the substrate was measured from a decrease in absorbency at a particular λmax of each of the substrates.

A blank test was conducted in substantially the same manner as above by adding 0.2 ml of the sodium phosphate buffer solution (pH 7.1) to 1.2 ml of the above substrate.

(2) Measurement for Stability of Test Compounds against DHP-I by High Performance Liquid Chromatography Method (HPLC):

The test compound according to the present invention and the control compounds were treated in substantially the same manner as (1) above. The incubation, however, was conducted at 37°C for 4.5 hours or for 24 hours. The degree of hydrolysis of the compounds each after the test periods was measured by the HPLC method.

3. Results:

The initial velocity of hydrolysis of each of the substrates against DHP-I by the late assay was found as follows:
Gl-dh-Ph: 17.4 µM/minute
Imipenem: 0.56 µM/minute

Table 3 below shows measurement results on stability of the compound according to the present invention and imipenem against DHP-I.

TABLE 3

DEGREES OF HYDROLYSIS BY DHP-I

(Method: HPLC; Substrate Concentration: 100 µM; Unit: µM)

| Incubation Conditions | Test Compounds: Imipenem | Test Compounds: Compound (15) |
|---|---|---|
| 37°C, 4.5 hours | 77.6 | 2.8 |
| 37°C, 24 hours | * | 2.9 |

* After 24 hours at 37°C, it has been found that most or all imipenem has been decomposed and nothing remained was detected.

The stability test results against DHP-I clearly show that the carbapenem compound according to the present invention was approximately twenty-eight times as stable imipenem.

V. Toxicity:

Toxicological studies were carried out using a group of 10 male mice of CrjCD(SD) strain weighing from 20 to 23 grams. A solution containing the carbapenem compound (15) of the present invention obtained by Example 6 was administered subcutaneously to the mice and subjected to observations for one week.

The results have revealed that the group of mice to which the carbapenem compound (15) of the present invention had been administered in the amount of 500 mg/kg were alive without any abnormal observations.

As described above, the carbapenem compounds according to the present invention demonstrate a wider scope of antibacterial spectra than do conventional cephalosporin compounds, and remarkable antibacterial activities comparable to imipenem as well as an overwhelmingly higher resistance against DHP than imipenem. The carbapenem compounds according to the present invention further possess antibacterial activities against clinically isolated strains and present favorable effects on infection preventive tests on mice against various organisms.

Therefore, the carbapenem compounds of formula (I) according to the present invention permit a single administration without combination with any other compounds and without a risk of any side effect that might be caused in their combined use with a DHP inhibitor, unlike imipenem that was led for the first time to a practically useful antibacterial agent in combination with cilastatin acting as a DHP inhibitor. The carbapenem compounds are accordingly extremely useful as antibacterial agents for therapy and prevention of infectious diseases from various pathogenic organisms.

The carbapenem compound of formula (I) according to the present invention may be administered as an antibacterial agent to the human being and other mammalian animals in the form of a pharmaceutically acceptable composition containing an antibacterially effective amount thereof. The administration dose may vary in a wide range with ages, patients, weights and conditions of patients, forms or routes of administration, physicians' diagnoses or the like and may be orally, parenterally or topically administered, to adult patients usually in a standard daily dose range from approximately 200 to approximately 3,000 mg once or in several installments per day.

The pharmaceutically acceptable composition of the carbapenem compound of formula (I) according to the present invention may contain an inorganic or organic, solid or liquid carrier or diluent, which is conventionally used for preparations of medicines, particularly antibiotic preparations, such as an excipient, e.g., starch, lactose, white sugar, crystalline cellulose, calcium hydrogen phosphate or the like; a binder, e.g., acacia, hydroxypropyl cellulose, alginic acid, gelatin, polyvinyl pyrrolidone or the like; a lubricant, e.g., stearic acid, magnesium stearate, calcium stearate, talc, hydrogenated plant oil or the like; a disintegrator, e.g., modified starch, calcium carboxymethyl cellulose, low substituted hydroxypropyl cellulose or the like; or a dissolution aid, e.g., a non-ionic surface active agent, an anionic surface active agent or the like, and may be prepared into forms suitable for oral, parenteral or topical administration. The formulations for oral administration may include solid preparations such as tablets, coatings, capsules, troches, powders, fine powders, granules, dry syrups or the like or liquid preparations such as syrups or the like; the formulations for parenteral administration may include, for example, injectable solutions, drip-feed solutions, depositories or the like; and the formulations for topical administration may include, for example, ointments, tinctures, creams, gels or the like. These formulations may be formed by procedures known per se to those skilled in the art in the field of pharmaceutical formulations.

The carbapenem compounds of formula (I) according to the present invention are suitably administered in the form of parenteral formulations, particularly in the form of injectable solutions.

The production of the carbapenem compounds of the formula (I) according to the present invention will be described more in detail by way of working examples.

In the following description, the following symbols are used to have the particular meanings.

ph : phenyl group

| | | |
|---|---|---|
| ph | : | phenyl group |
| PNB | : | p-nitrobenzyl group |
| PNZ | : | p-nitrobenzyloxycarbonyl group |
| ⅄Si | : | tertiary-butyldimethylsilyl group |
| Ac | : | acetyl group |
| Et | : | ethyl group |

Example 1:

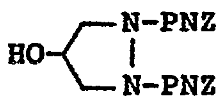

Compound (1)

To 15 g of hydrazine monohydrate in a 50 ml flask was added dropwise 9.3 g of epichlorohydrin at 0°C over 1 hour. After addition, the reaction mixture was stirred for 2 hours at the above temperature. After removal of the excess hydrazine under reduced pressure, 300 ml of a saturated sodium bicarbonate solution and 200 ml of tetrahydrofuran were added to the residue. To this solution was added dropwise a solution of 43 g of p-nitrobenzyloxycarbonyl chloride in 150 ml of tetrahydrofuran. The reaction mixture was stirred for 2 hours. After the reaction, 200 ml of ethyl acetate was added to the reaction mixture. The organic layer was separated and the water layer was extracted with 100 ml of ethyl acetate. The organic layers were combined and washed with a saturated sodium chloride aqueous solution and dried over sodium sulfate. The solvent was removed and 500 ml of chloroform was added to the residue. The resulting solution was stored in a refrigerater to give a precipitate. After removal of the precipitate, the solvent was removed and the residue was purified by silica gel column chromatography (dichloromethane) to give 16.7 g of Compound (1).
NMR ($CDCl_3$) δ : 8.15 (4H, d), 7.48 (4H, d) 5.4-5.0 (1H, m), 5.37 (4H, s), 4.4-3.2 (4H, m).

Example 2:

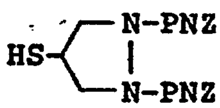

Compound (2)

(a) To a solution of 16.6 g of Compound (1) obtained by Example 1 and 5.6 g of triethylamine in 200 ml of dichloromethane was added dropwise a solution of 6.42 g of methanesulfonyl chloride in 20 ml of dichloromethane at 0°C, and the reaction mixture was stirred for 15 minutes at room temperature. After the reaction, the organic layer was washed with 200 ml of water, 200 ml of a saturated sodium bicarbonate solution and 200 ml of a saturated sodium chloride solution and then dried over sodium sulfate. The solvent was removed to give 16.6 g of a pale yellowish powder.

(b) A solution of 9.6 g of the above powder, 3.13 g of potassium acetate and 250 ml of acetone was refluxed for 2 hours. After addition of 50 ml of water, the reaction solvent was removed and the resulting residue was extracted with ethyl acetate. The organic layer was washed with water, dried over sodium sulfate and removed. The resulting residue was purified using silica gel column chromatography by chloroform as an eluent to give 5.9 g of powder.

(c) To a solution of 13.6 g of the above powder, 145 g of tetrahydrofuran and 145 ml of methanol was added 12.3 ml of 4% sodium methoxide-methanol solution at 0°C and the reaction mixture was stirred for 5 minutes. After the reaction, 1N-hydrogen chloride solution was added to the reaction mixture and the resulting acidic solution was extracted with ethyl acetate. After washing and drying, the solvent was removed to give 11.9 g (63%) of Compound (2) as pale brownish powder.
NMR ($CDCl_3$) δ : 8.17 (4H, d), 7.48 (4H, d), 5.28 (4H, s), 4.5-3.2 (5H, m).

Example 3:

(A)

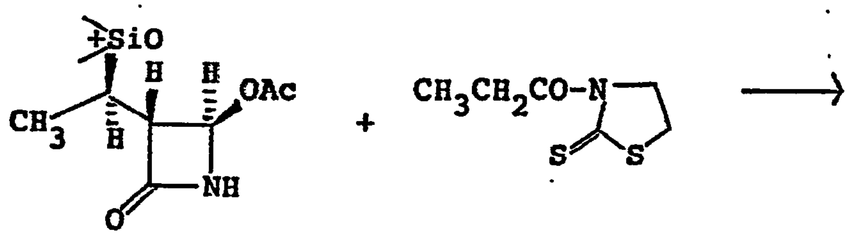

(3) (4)

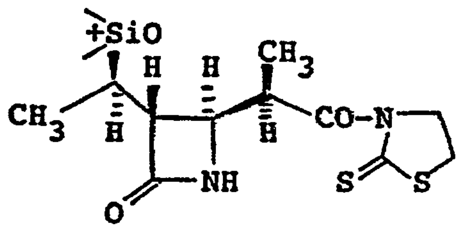

(5)

Tin triflate (3.712 g) was dissolved in 10 ml of anhydrous tetrahydrofuran under a nitrogen gas stream, and the resulting solution was cooled to 0°C. To this solution were added 1.3 ml of N-ethylpiperidine and a solution of 1.2 g of Compound (4) above in 7 ml of anhydrous tetrahydrofuran. The mixture was stirred for 2 hours at the above temperature. A solution of 1.42 g of Compound (3) in 2 ml of anhydrous tetrahydrofuran was added, and the resultant mixture was stirred for 1 hour. After the completion of the reaction, 100 ml of chloro form was added and the mixture was washed with a 10% cirtic acid aqueous solution. The organic layer separated was then dried over $MgSO_4$ and the solvent was removed. The residue was purified by silica gel column chromatography with a n-hexane:ethyl acetate (2-1:1) mixture to give 1.93 g (97%) of Compound (5) as a yellow solid.

NMR ($CDCl_3$) δ : 0.07 (6H, s), 0.88 (9H, s), 1.21 (3H, d), 1.26 (3H, d), 3.30 (1H, dd), 3.38 (2H, t), 3.94 (1H, dd), 4.55 (2H, t), 6.24 (1H, bs).

(B)

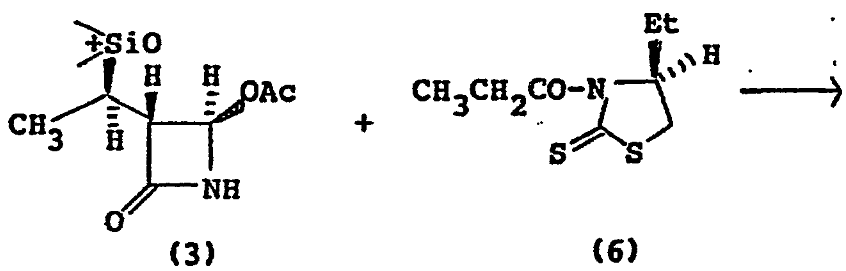

(3) (6)

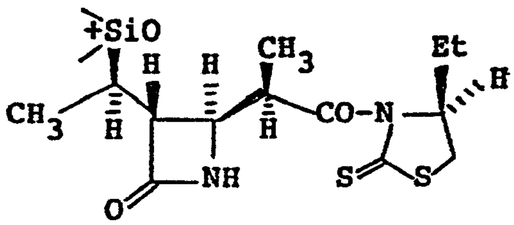

(7)

Tin triflate (57.0 g) was dissolved in 164 ml of anhydrous tetrahydrofuran under a nitrogen gas stream, and the resulting solution was cooled to 0°C. To this solution were added 19.9 ml of N-ethylpiperidine and a solution of 21.71 g of Compound (6) above in 123 ml of anhydrous tetrahydrofuran. The mixture was stirred for 1.5 hours at the above temperature. A solution of 1.42 g of Compound (3) in 123 ml of anhydrous tetrahydrofuran was added, and the resultant mixture was stirred for 1 hour. After the completion of the reaction, chloroform was added and the mixture was washed with a 10% citric acid aqueous solution and a sodium chloride aqueous solution. The organic solution separated was then dried over $MgSO_4$ and the solvent was removed. The residue was purified by silica gel column chromatography with n-hexane:ethyl acetate (2:1) to give 33.57 g (98%) of Compound (7) as a yellow solid, m.p. 85.5-86.5°C.
NMR ($CDCl_3$) δ : 0.07 (6H, s), 0.90 (9H, s), 1.00 (3H, t), 1.23 (3H, d), 1.26 (3H, d), 2.90 (1H, dd), 3.50 (1H, dd), 6.10 (1H, bs).
$[\alpha]_D^{25}$ = +233.9° (c=0.77, $CHCl_3$).

(C)

Compound (7) 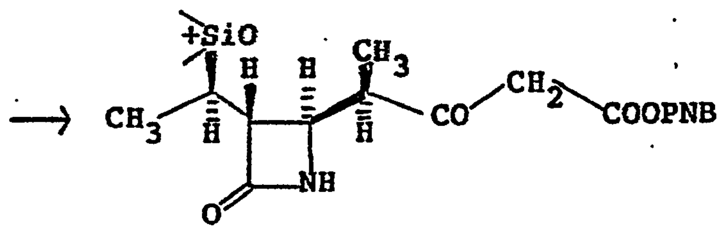

(8)

To a solution of 30.66 g of Compound (7) obtained in the step (B) above in 740 ml of anhydrous acetonitrile was added 12.13 g of imidazole, and the mixture was stirred under a nitrogen gas stream at room temperature for 5.5 hours. Then, 53.39 g of $Mg(O_2CCH_2CO_2PNB)_2$ was added, and the mixture was stirred overnight at 60°C. The resultant reaction mixture was concentrated under reduced pressure to 200 ml and 1 liter of ethyl acetate was added thereto. The organic layer separated was washed with a 1N-HCl aqueous solution, a 5% aqueous $NaHCO_3$ solution and an aqueous sodium chloride solution in this order. After drying over $MgSO_4$, the solvent was removed and the residue was purified by column chromatography with 800 g of silica gel to yield 34.47 g of Compound (8) as a colorless oil.
NMR ($CDCl_3$) δ : 0.06 (6H, s), 0.87 (9H, s), 1.16 (3H, d), 1.20 (3H, d), 3.63 (2H, s), 5.27 (2H, s), 5.92 (1H, bs), 7.56, 8.24 (4H aromatic ring proton)
Compound (8) obtained above was used in the following step (D) without further purification.

(D)

Compound (8) 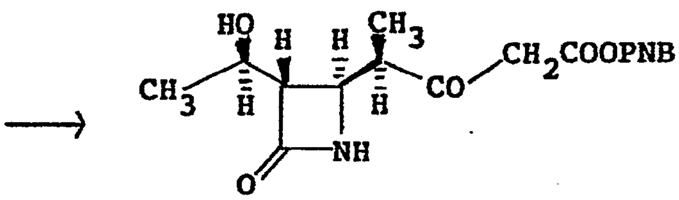

(9)

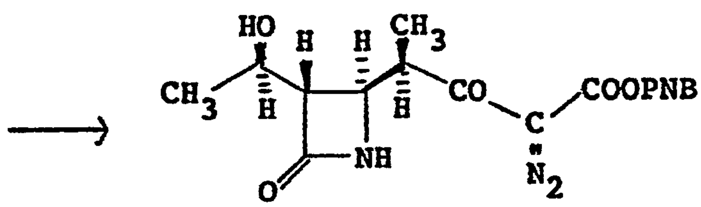

(10)

To a solution of 37.47 g of Compound (8) obtained in the step (C) above in 392 ml of methanol was added 19.6 ml of concentrated HCl, and the mixture was stirred at room temperature for 1.5 hours. The reaction mixture was concentrated to approximately 100 ml, and 800 ml of ethyl acetate was added. After the mixture was washed with water and then wtih an aqueous sodium chloride slution, it was then dried over $MgSO_4$. The solvent was removed under reduced pressure to yield Compound (9) as a colorless oil.
NMR ($CDCl_3$) δ : 1.25 (3H, d), 1.30 (3H, d), 2.90 (2H, m), 2.65 (2H, s), 3.83 (1H, m), 4.15 (1H, m), 5.27 (2H, s), 6.03 (1H, bs), 7.55, 8.27 (4H, aromatic ring proton)

Compound (9) was then dissolved in 408 ml of anhydrous acetonitrile, and 36.31 g of dodecylbenzyl sulfonyl azide and 13.81 ml of triethylamine were added. After the mixture was stirred at room temperature for 20 minutes, the solvent was removed. The residue was purified by means of column chromatography with 800 g of silica gel using chloroform:acetone (2:1) to give 21.57 g [69.4% as total yields of Compounds (B), (C) and (D)] of Compound (10) as a colorless oil.
IR ($CHCl_3$) $cm^{-1}$: 2150, 1750, 1720, 1650.
NMR ($CDCl_3$) δ : 1.23 (3H, d), 1.30 (3H, d), 2.92 (1H, m), 3.50-4.30 (3H, m), 5.38 (2H, s), 6.40 (1H, bs), 7.57, 8.30 (4H, aromatic ring proton)
$[\alpha]_D^{21}$ = -41.6° (c=3.1, $CH_2Cl_2$).

(E)

Compound (10)

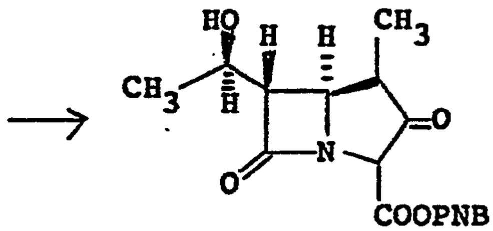

(11)

In 134 ml of ethyl acetate was dissolved 21.57 g of Compound (10) obtained in the step (D) above, and 0.065 g of rhodium octanoate was added. The solution was stirred at 80°C for 0.5 hour and the solvent was removed. The residue was dried to give Compound (11) as a solid.
IR ($CHCl_3$) $cm^{-1}$: 2950, 2925, 1860, 1830.
NMR ($CDCl_3$) δ : 1.22 (3H, d, J=8.0Hz), 1.37 (3H, d, J=6.0Hz), 2.40 (1H, bs), 2.83 (3H, q, J=8.0Hz), 3.28 (1H, d, d), 4.00-4.50 (2H, m), 4.75 (1H, s), 5.28 and 5.39 (2H, ABq, J=12Hz), 7.58, 8.24 (4H, aromatic ring proton)

(F)

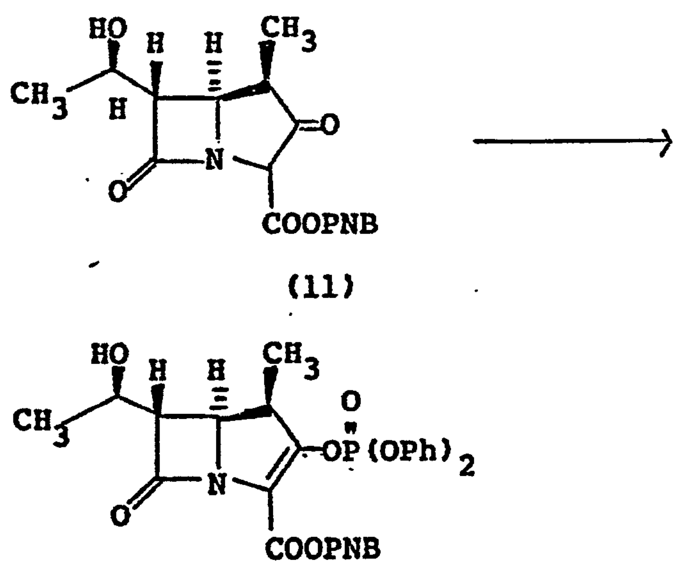

(11)

(12)

To a solution of 186 mg of Compound (11) obtained in the step (E) in 2 ml of anhydrous acetonitrile were added 0.11 ml of diphenylphosphoric chloride and 0.09 ml of diisopropylethyl amine under cooling with ice, and the mixture was stirred for 0.5 hour at the same temperature. After the reaction mixture was concentrated, the residue was purified by silica gel column chromatography to yield 252 mg of Compound (12) as a white solid.
NMR ($CDCl_3$) δ : 1.24 (3H, d), 1.34 (3H, d), 3.30 (1H, q), 3.52 (1H, m), 4.10-4.40 (2H, m), 5.20 and 5.35 (2H, q), 7.29 (1H, m), 7.58 and 8.18 (4H, d).

Example 4:

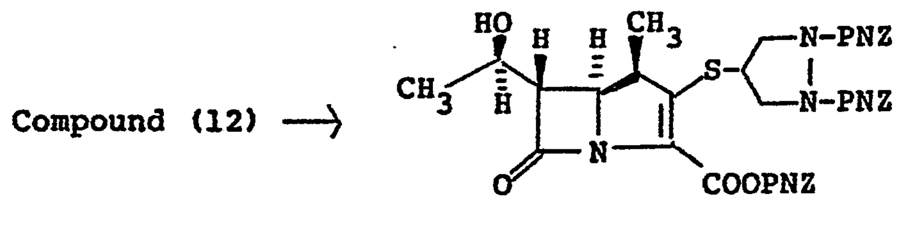


(13)

To a solution of 476 mg of Compound (12) obtained in Example 3 in anhydrous acetonitrile was added a solution of 460 mg of Compound (2) obtained in Example 2 and 0.17 ml of diisopropylethyl amine, and the mixture was stirred for 40 minutes under a nitrogen atmosphere. Removal of the solvent left a residue that was in turn purified by means of silica gel column chromatography (chloroform:acetone = 3:1) to yield 667 mg (100%) of Compound (13).
NMR ($CDCl_3$) δ : 1.24 (3H, d, J=6.0Hz), 1.35 (3H, d, J=6.0Hz), 3.2-4.9 (9H, m), 5.16 (1H, d, J=15.0Hz), 5.26 (2H, s), 5.47 (1H, d, J=15.0Hz), 7.3-7.7 (6H, m), 0.85-8.3 (6H, s).

Example 5:

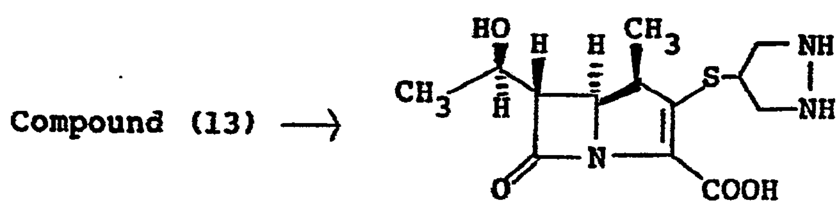


(14)

To a solution of 667 mg of Compound (13) in 7 ml of tetrahydrofuran and 7 ml of water was added 120 mg of platinum oxide, and the catalytic hydrogenation was carried out at room temperature for 1 hour under a pressure of 3.0 atmospheres. After removal of the catalyst, the solvent was removed to give 192 mg (74.0%) of Compound (14) after lyophilization.
IR (KBr) $cm^{-1}$: 1750.
NMR ($D_2O$-$CD_3OD$) δ: 1.23 (3H, d, J=6.0Hz), 1.40 (3H, d, J=7.0Hz), 3.3-4.4 (9H, m).

Example 6:

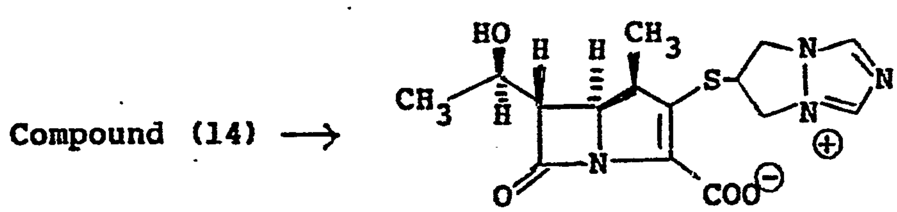


(15)

192 mg of Compound (14) was dissolved in 15 ml of phosphate buffer solution (pH 7.0), and the pH of the solution was adjusted to 8.5 by 1N sodium hydroxide solution. To this solution was added 570 mg of ethyl

formimidate hydrochloride, and the reaction mixture was stirred for 1 hour under ice-cooling. After the pH of the reaction mixture was adjusted to 7.0, the solvent was removed and the resulting residue was lyophilized. The resulting powder was purified using HP-40 column (water, 3% acetone-water) to give 76 mg (33.8%) of (1R,5S,6S)-2-[(6,7-dihydro-5H-pyrazolo[1,2-a][1,2,4]-triazolium-6-yl)]thio-6-[(R)-1-hydroxyethyl]-1-methyl-carbapenem-3-carboxylate [Compound (15)] after lyophilization.
NMR ($D_2O$) δ: 1.32 (3H, d, J=6.0Hz), 1.40 (3H, d, J=6.0Hz), 3.3-4.4 (9H, m), 9.12 (2H, s).

Example 7:

Crystalline (1R,5S,6S)-2-[(6,7-dihydro-5H-pyrazolo[1,2-a][1,2,4]-triazolium-6-yl)[thio-6-[(R)-1-hydroxyethyl]-1-methyl-carbapenem-3-carboxylate [Crystalline Compound (15)]:

45 mg of (1R,5S,6S)-2-[(6,7-dihydro-5H-pyrazolo[1,2-a][1,2,4]-triazolium-6-yl)]thio-6-[(R)-1-hydroxyethyl]-1-methyl-carbapenem-3-carboxylate was dissolved in 4 ml water. The resultant solution was filtered using a Membran® filter (0.22 μm). The filtrate was lyophilized to give amorphous (1R,5S,6S)-2-[(6,7-dihydro-5H-pyrazolo[1,2-a][1,2,4]-triazolium-6-yl)]thio-6-[(R)-1-hydroxyethyl]-1-methyl-carbapenem-3-carboxylate. This amorphous form of (1R,5S,6S)-2-[(6,7-dihydro-5H-pyrazolo[1,2-a][1,2,4]-triazolium-6-yl)]thio-6-[(R)-1-hydroxyethyl]-1-methyl-carbapenem-3-carboxylate was then again dissolved in 0.4 ml of water, and the resultant solution warmed to 40°C to completely dissolve the (1R,5S,6S)-2-[(6,7-dihydro-5H-pyrazolo-[1,2-a][1,2,4]-triazolium-6-yl)]thio-6-[(R)-1-hydroxyethyl]-1-methyl-carbapenem-3-carboxylate. After storage under refrigeration conditions for a period of 3 hours, the sample was inspected and crystals were observed. The crystalline (1R,5S,6S)-2-[(6,7-dihydro-5H-pyrazolo[1,2-a][1,2,4]-triazolium-6-yl)]thio-6-[(R)-1-hydroxyethyl]-1- methyl-carbapenem-3-carboxylate was washed with a small amount of 50% ethanolic solution in water, and the resultant crystals were dried at room temperature under a vacuum to yield 34 mg of crystalline (1R,5S,6S)-2-[(6,7-dihydro-5H-pyrazolo[1,2-a][1,2,4]-triazolium-6-yl)]thio-6-[(R)-1-hydroxyethyl]-1-methyl-carbapenem-3-carboxylate (75.6%).

This crystalline product was in the form of colorless fine needle with a melting point of 215°C (decomp.), and showed characteristic peaks in planar spacings (d) 5.8, 5.5, 4.9, 4.8, 4.4, 4.3, 4.0, 3.5, 3.3, 3.2 and 3.0 in an X-ray diffraction pattern.

The carbapenem compounds according to the present invention may be formulated in various preparation forms.

**Formulation Example 1 (Injection):**

**(1) Injectable suspension:**

| | |
|---|---|
| Compound (15) | 25.0 g |
| Methyl cellulose | 0.5 g |
| Polyvinyl pyrrolidone | 0.05 g |
| Methyl p-oxybenzoate | 0.1 g |
| Polysolvate 80 | 0.1 g |
| Lidocaine hydrochloride | 0.5 g |
| Distilled water | to make 100 ml |

The above components were formulated into 100 ml of an injectable suspension.

(2) Lyophilization:

An appropriate amount of distilled water was added to 20 g of the sodium salt of Compound (15) to make a total volume of 100 ml. The above solution (2.5 ml) was filled in vials so as for each vial to contain 500 mg of the sodium salt of Compound (15) and lyophilized. The lyophilized vial was mixed in situ with approximately 3-4 ml of distilled water to make an injectable solution.

(3) Powder:

Compound (15) was filled in an amount of 250 ml in a vial and mixed in situ with about 3-4 ml of distilled water to make an injectable solution.

Formulation Example 2 (Tablets):

| | |
|---|---|
| Compound (15) | 250 mg |
| Lactose | 250 mg |
| Hydroxypropyl cellulose | 1 mg |
| Magnesium stearate | 10 mg |
| | 511 mg/tablet |

The above components were mixed with each other and punched into tablets in conventional manner. Such tablets, as required, may be formulated into sugar coatings or film coatings in conventional manner.

Formulation Example 3 (Troche):

| | |
|---|---|
| Compound (15) | 200 mg |
| Sugar | 770 mg |
| Hydroxypropyl cellulose | 5 mg |
| Magnesium stearate | 20 mg |
| Flavor | 5 mg |
| | 1,000 mg/troche |

The component was mixed with each other and formulated into troches by punching in conventional manner.

Formulation Example 4 (Capsulas):

Formulation Example 4 (Capsules):

| | |
|---|---|
| Compound (15) | 500 mg |
| Magnesium stearate | 10 mg |
| | 510 mg/capsule |

The component was mixed with each other and filled in conventional hard gelatin capsules.

Formulation Example 5 (Dry Syrup):

| | |
|---|---|
| Compound (15) | 200 mg |
| Hydroxypropyl cellulose | 5 mg |
| Sugar | 793 mg |
| Flavor | 5 mg |
| | 1,000 mg |

The above components were mixed with each other and formulated into dry syrups in conventional manner.

Formulation Example 6 (Powders):

| | | |
|---|---|---|
| (1) | Compound (15) | 200 mg |
| | Lactose | 800 mg |
| | | 1,000 mg |
| (2) | Compound (15) | 250 mg |
| | Lactose | 750 mg |
| | | 1,000 mg |

Each of the components was mixed with each other and formulated in powders in conventional manner.

Formulation Example 7 (Suppository):

| | |
|---|---|
| Compound (15) | 500 mg |
| Witepsol H-12 | 700 mg |
| (Product of Dynamite Noble) | |
| | 1,200 mg |

The above components were mixed with each other and formulated into suppositories in conventional manner.

**Claims for the Contracting States: AT, BE, CH, DE, FR, GB, IT, LI, LU, NL, SE**

1. A (1R,5S,6S)-2-substituted thio-6-[(R)-1-hydroxyethyl]-1-methyl-carbapenem-3-carboxylic acid derivative represented by the following formula

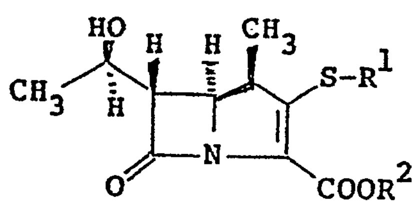

(I)

wherein $R^1$ represents a radical of the formula

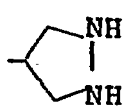

or

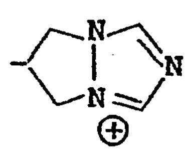

and $R^2$ is a hydrogen atom or an anion charge, or a pharmacologically acceptable salt thereof.

2. The compound of claim 1 which is (1R,5S,6S)-2-[4-pyrazolidinyl]-thio-6-[(R)-1-hydroxyethyl]-1-methyl-carbapenem-3-carboxylic acid of the following formula

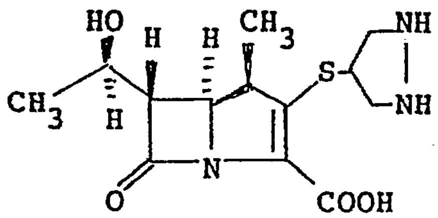

(I-1)

or a pharmacologically acceptable salt thereof.

3. The compound of claim 1 which is (1R,5S,6S)-2-[(6,7-dihydro-5H-pyrazolo[1,2-a][1,2,4]-triazolium-6-yl)]thio-6-[(R)-1-hydroxyethyl]-1-methyl-carbapenem-3-carboxylate of the following formula

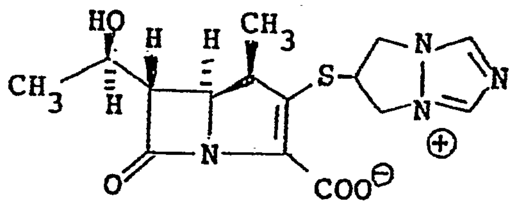

(I-2)

or a pharmacologically acceptable salt.

4. The compound of claim 3 which is the compound of formula (I-2) in a crystalline form, or a pharmacologically acceptable salt.

5. The compound of any one of claims 1 to 4 as a pharmaceutically active substance.

6. (1R,5S,6S)-2-[(6,7-dihydro-5H-pyrazolo[1,2-a][1,2,4]triazolium-6-yl)]thio-6-[(R)-1-hydroxyethyl]-1-methyl-carbapenem-3-carboxylate or a pharmacologically acceptable salt thereof as a pharmaceutically active substance.

7. The compound of any one of claims 1 to 4 as an antibacterial agent.

8. (1R,5S,6S)-2-[(6,7-dihydro-5H-pyrazolo[1,2-a][1,2,4]triazolium-6-yl)]thio-5-[(R)-1-hydroxyethyl]-1-methyl-carbapenem-3-carboxylate or a pharmacologically acceptable salt thereof as an antibacterial agent.

9. A process for producing a (1R,5S,6S)-2-substituted thio-6-[(R)-1-hydroxyethyl]-1-methyl-carbapenem-3-carboxylic acid derivative represented by the following formula

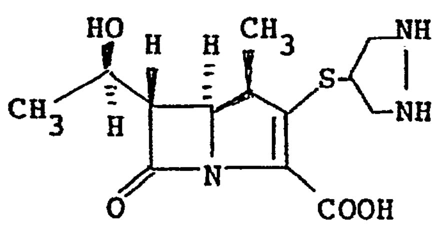

(I-1)

or a pharmacologically acceptable salt thereof; which comprises reacting a compound represented by the following formula

3z

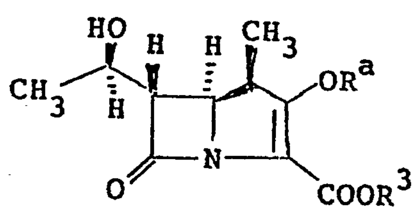

(II)

wherein $R^3$is a carboxyl protecting group, and $R^a$ is an acyl group, with a mercapto reagent represented by the following formula

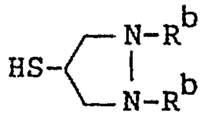

(III)

wherein $R^b$ is an amino protecting group, to give a compound represented by the following formula

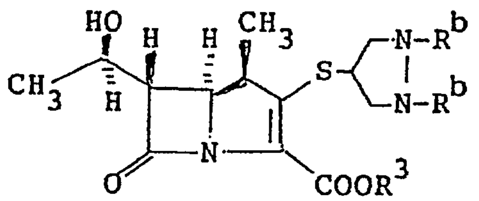

(IV)

wherein $R^3$ and $R^b$ have the same meanings as above,
and subjecting the compound of the formula (IV) to removal of the protecting groups $R^3$ and $R^b$.

10. Process for producing (1R,5S,6S)-2-[(6,7-dihydro-5H-pyrazolo [1m2-a] [1,2,4] triazolium-6-yl)] thio-6-[(R)-1-hydroxyethyl]-1-methyl-carbapenem-3-carboxylate represented by the following formula

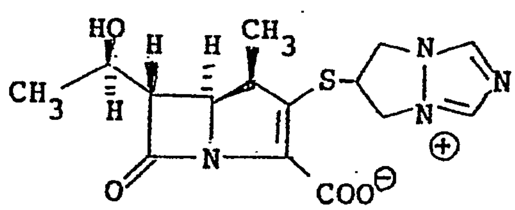

(I-2)

or a pharmacologically acceptable salt thereof; which comprises reacting a compound represented by the following formula

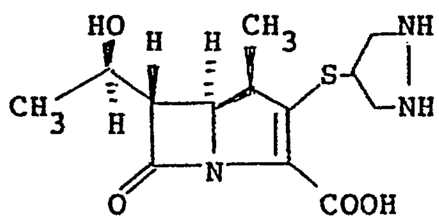

(I-1)

with formimidic acid ester.

11. A medicament comprising (1R,5S,6S)-2-[(6,7-dihydro-5H-pyrazolo[1,2-a][1,2,4]triazolium-6-yl)]thio-6-[(R)-1-hydroxyethyl]-1-methyl-carbapenem-3-carboxylate or a pharmacologically acceptable salt thereof.

12. An antibacterial agent comprising (1R,5S,6S)-2-[(6,7-dihydro-5H-pyrazolo[1,2-a][1,2,4]triazolium-6-yl)]thio-6-[(R)-1-hydroxyethyl]-1-methyl-carbapenem-3-carboxylate or a pharmacologically acceptable salt thereof.

13. The use of the compound according to any one of claims 3 or 4 for the manufacture of antibacterial medicaments.

14. The use of the compound according to any one of claims 1 to 4 for controlling or preventing a disease.

15. The use of the compound according to any one of claims 1 to 4 for controlling or preventing a bacterial infection.

16. The use of the compound according to any one of claims 1 to 4 for the manufacture of antibacterial medicaments.

**Claims for the Contracting State: ES**

1. Process for producing (1R,5S,6S)-2-[4-pyrazolidinyl]-thio-6-[(R)-1-hydroxyethyl-]-1-methyl-carbapenem-3-carboxylic acid represented by the following formula

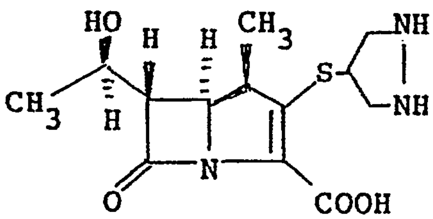

(I-1)

or a pharmacologically acceptable salt thereof; which comprises reacting a compound represented by the following formula

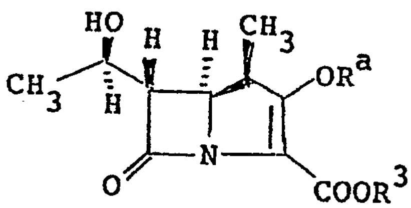

(II)

wherein $R^3$ is a carboxyl protecting group, and $R^a$ is an acyl group, with a mercapto reagent represented by the following formula

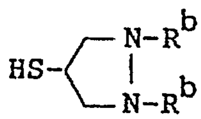

(III)

wherein $R^b$ is an amino protecting group, to give a compound represented by the following formula

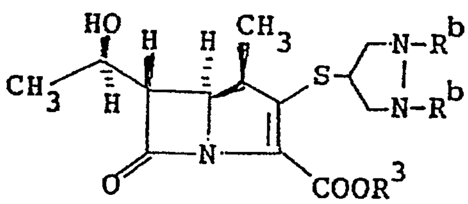

(IV)

wherein $R^3$ and $R^b$ have the same meanings as above, and subjecting the compound of the formula (IV) to removal of the protecting groups $R^3$ and $R^b$.

2. Process for producing (1R,5S,6S)-2-[(6, 7-dihydro-5H-pyrazolo[1, 2-a] [1,2,4] triazolium-6-yl)] thio-6-[(R)-1-hydroxyethyl]-1-methyl-carbapenem-3-carboxylate represented by the following formula

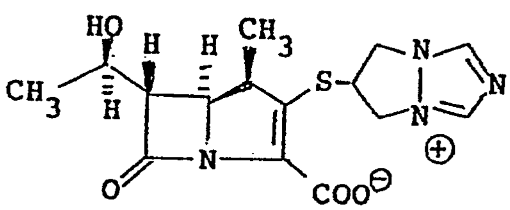

(I-2)

or a pharmacologically acceptable salt thereof; which comprises reacting a compound represented by the following formula

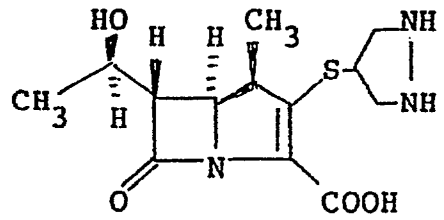

(I-1)

with formimidic acid ester.

3. The process of claim 1 wherein the product (1R,5S,6S)-2-[4-pyrazolidinyl]-thio-6-[(R)-1-hydroxyethyl]-1-methyl-carbapenem-3-carboxylic acid of the following formula

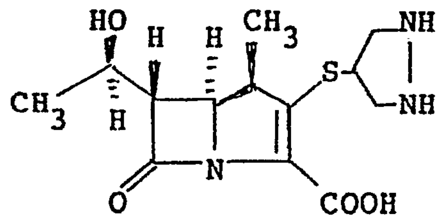

(I-1)

or a pharmacologically acceptable salt thereof ist obtained.

4. The process of claim 2 wherein the product (1R,5S,6S)-2-[6, 7-dihydro-5H-pyrazolo[1, 2-a] [1,2,4]-triazolium-6-yl] thio-6-[(R)-1-hydroxyethyl]-1-methyl-carbapenem-3-carboxylate of the following formula

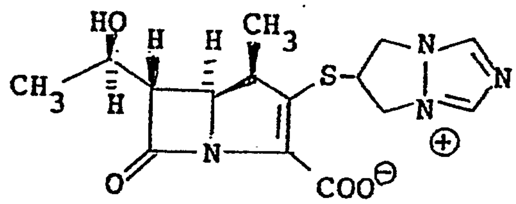

(I-2)

or a pharmacologically acceptable salt is obtained.

5. The process of claim 4 wherein the compound of formula (I-2) in a crystalline form, or a pharmacologically acceptable salt is obtained.

6. The process according to claim 2 wherein the product (1R,5S,6S)-2-[(6, 7-dihydro-5H-pyrazolo[1, 2-a][1,2,4] triazolium-6-yl)] thio-6-[(R)-1-hydroxyethyl]-1-methyl-carbapenem-3-carboxylate or a pharmacologically acceptable salt thereof as a pharmaceutically active substance is obtained.

7. The process according to claim 2 wherein the product (1R,5S,6S)-2-[(6, 7-dihydro-5H-pyrazolo[1, 2-a][1,2,4] triazolium-6-yl)] thio-6-[(R)-1-hydroxyethyl]-1-methyl-carbapenem-3-carboxylate or a pharmacologically acceptable salt thereof as an antibacterial agent is obtained.

8. A process for preparing an antibacterial agent comprising mixing (1R,5S,6S)-2-[(6, 7-dihydro-5H-pyrazolo[1, 2-a][1,2,4] triazolium-6-yl)] thio-6-[(R)-1-hydroxyethyl]-1-methyl-carbapenem-3-carboxylate or a pharmacologically acceptable salt thereof and a pharmacologically acceptabel carrier and/or diluent.

9. A process for preparing an antibacterial agent comprising mixing (1R,5S,6S)-2-[(6, 7-dihydro-5H-pyrazolo[1, 2-a][1,2,4] triazolium-6y1)] thio-6-[(R)-1-hydroxyethyl]-1-methyl-carbapenem-3-carboxylate or a pharmacologically acceptable salt thereof and a pharmacologically acceptabel carrier and/or diluent.

Claims for the Contracting State: GR

1. A (1R,5S,6S)-2-substituted thio-6-[(R)-1-hydroxyethyl]-1-methyl-carbapenem-3-carboxylic acid derivative represented by the following formula

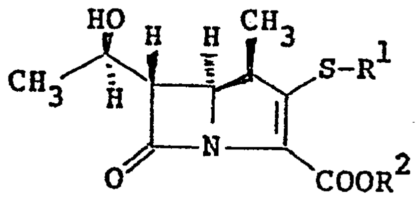

(I)

wherein $R^1$ represents a radical of the formula

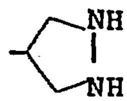

or

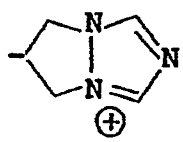

and $R^2$ is a hydrogen atom or an anion charge or a salt thereof.

2. The compound of claim 1 which is (1R,5S,6S)-2-[4-pyrazolidinyl]-thio-6-[(R)-1-hydroxyethyl]-1-methyl-carbapenem-3-carboxylic acid of the following formula

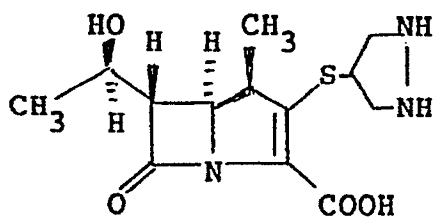

(I-1)

or salt thereof.

3. The compound of claim 1 which is (1R,5S,6S)-2-[(6,7-dihydro-5H-pyrazolo[1,2-a] [1,2,4]-triazolium-6-yl)] thio-6-[(R)-1-hydroxyethyl]-1-methyl-cyrbapenem-3-carboxylate of the following formula

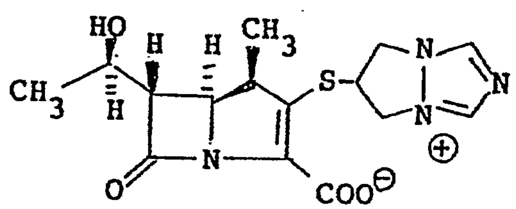

(I-2)

or a salt thereof.

4. The compound of claim 3 which is the compound of formula (I-2) in a crystalline form, or a salt thereof.

5. The compound of any one of claims 3 or 4 as an antibacterial agent.

6. Use of (1R,5S,6S)-2-[(6,7-dihydro-5H-pyrazolo[1,2-a] [1,2,4]triazolium-6-yk)]thio-6-[(R)-1-hydroxyethyl]-1-methyl-carbapenum-3-carboxylate or a pharmacologically acceptable salt thereof as an antibacterial agent.

7. Process for producing (1R,5S,6S)-2-[4-pyrazolidinyl]-thio-6-[(R)-hydroxyethyl]-1-methyl-carbapenum-3-carboxylic acid represented by the following formula

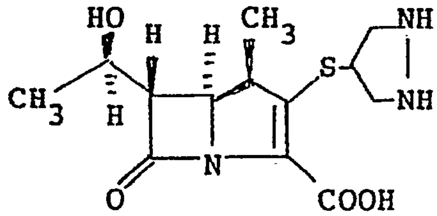

(I-1)

or a pharmacologically acceptable salt thereof; which comprises reacting a compound represented by the following formula

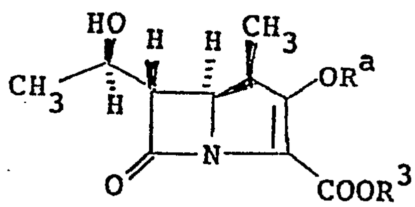

(II)

wherein $R^3$ is a carboxyl protecting group, and $R^a$ is an acyl group, with a mercapto reagent represented by the folllowing formula

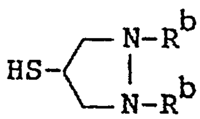

(III)

wherein $R^b$ is an amino protecting group, to give a compound represented by the following formula

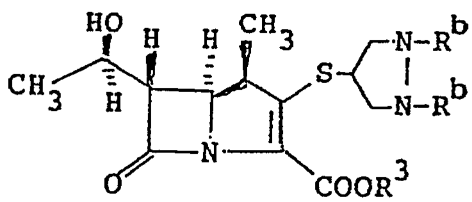

(IV)

wherein $R^3$ and $R^b$ have the same meanings as above, and subjecting the compound of the formula (IV) to removal of the protecting groups $R^3$ and $R^b$.

8. Process for producing (1R,5S,6S)-2-[(6,7-dihydro-5H-pyrazolo[1,2-a] [1,2,4]triazolium-6-yl)]thio-6-[(R)-1-hydroxyethyl]-1-methyl-carbapenem-3-carboxylate represented by the following formula

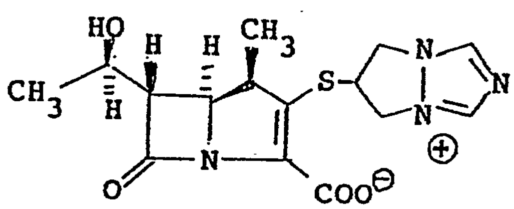

(I-2)

or a pharmacologically acceptable salt thereof; which comprises reacting a compound represented by the following formula

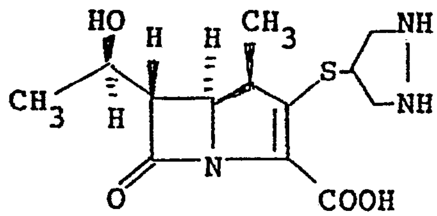

(I-1)

with formimidic acid ester.

9. A process for preparing a mixture comprising mixing (1R,5S,6S)-2-[(6,7-dihydro-5H-pyrazolo[1,2-a] [1,2,4]triazolium-6-yl)] thio-6-[(R)-1-hydroxyethyl]-1-methyl-carbapenem-3-carboxylate or a pharmacologically acceptable salt thereof and a pharmacologically acceptable carrier and/or diluent.

10. A process for preparing an antibacterial agent comprising mixing the (1R,5S,6S)-2-[(6,7-dihydro-5H-pyrazolo[1,2-a] [1,2,4]triazolium-6-yl)]-thio-6-[(R)-1-hydroxyethyl] 1-methyl-carbapenem-3-carboxylate or a pharmacologically acceptable salt thereof and a pharmacologically acceptable carrier and/or diluent.

11. The use of a compound according to any one of claims 3 or 4 for the manufacture of antibacterial agents.

**Patentansprüche für die benannten Vertragsstaaten: AT, BE, CH, DE, FR, GB, IT, LI, LU, NL, SE**

1. Ein (1R,5S,6S)-2-substituiertes Thio-6-[(R)-1-hydroxyethyl]-1-methyl-carbapenem-3-carbonsäurederivat, dargestellt durch die folgende Formel

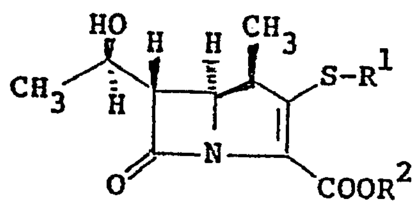

(I)

worin $R^1$ eine Gruppe der Formel

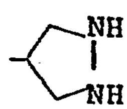

or

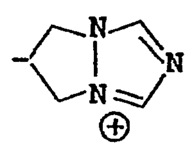

bedeutet und $R^2$ ein Wasserstoffatom oder eine anionische Ladung bedeutet, oder ein pharmakologisch annehmbares Salz davon.

2. Verbindung nach Anspruch 1, nämlich (1R,5S,6S)-2-[4pyrazolidinyl]-thio-6-[(R)-1-hydroxyethyl]-1-methyl-carbapenem-3-carbonsäure der folgenden Formel

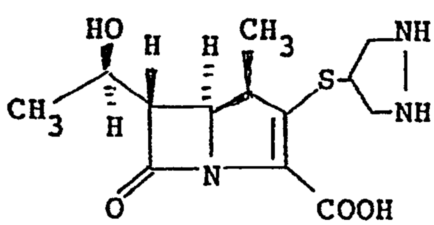

(I-1)

oder ein pharmakologisch annehmbares Salz davon.

3. Verbindung nach Anspruch 1, nämlich (1R,5S,6S)-2[(6, 7-Dihydro-5H-pyrazolo[1, 2-a][1,2,4]-triazolium-6-yl)]thio-6-[(R)-1-hydroxyethyl]-1-methyl-carbapenem-3-carboxylat der folgenden Formel

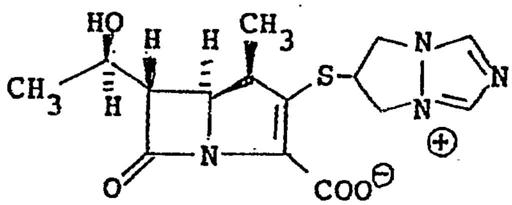

(I-2)

oder ein pharmakologisch annehmbares Salz davon.

4. Verbindung nach Anspruch 3, nämlich die Verbindung der Formel (I-2) in kristalliner Form, oder ein pharmakologisch annehmbares Salz davon.

5. Verbindung nach einem der Ansprüche 3 oder 4 als pharmazeutisch aktive Substanz.

6. (1R,5S,6S)-2-[(6, 7-Dihydro-5H-pyrazolo[1, 2-a][1,2,4]triazolium-6-yl)]thio-6-[(R)-1-hydroxyethyl]-1-methyl-carbapenem-3-carboxylat oder ein pharmakologisch annehmbares Salz davon als pharmazeutisch aktive Substanz.

7. Verbindung nach irgendeinem der Ansprüche 3 oder 4 als antibakterielles Mittel.

8. (1R,5S,6S)-2-[(6, 7-Dihydro-5H-pyrazolo[1, 2-a][1,2,4]triazolium-6-yl)]thio-6-[(R)-1-hydroxyethyl]-1-methyl-carbapenem-3-carboxylat oder ein pharmakologisch annehmbares Salz davon als antibakterielles Mittel.

9. Verfahren zur Herstellung von (1R,5S,6S)-2-[4-Pyrazolidinyl]-thio-6-[(R)-1-hydroxyethyl]-1-methyl-carbapenem-3-carbonsäure der folgenden Formel

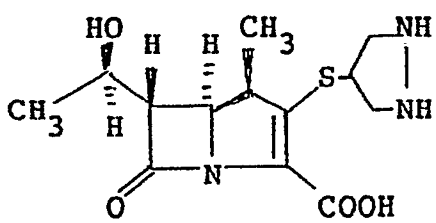

(I-1)

oder eines pharmakologisch annehmbaren Salzes davon; dadurch gekennzeichnet , daß eine Verbindung der folgenden Formel

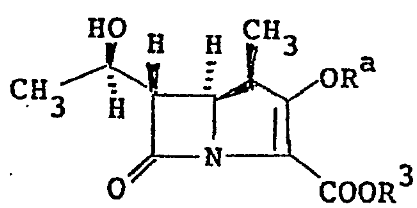

(II)

worin $R^3$ eine Carboxyl-Schutzgruppe und $R^a$ eine Acylgruppe bedeutet, mit einem Mercaptoreagens, dargestellt durch die folgende Formel

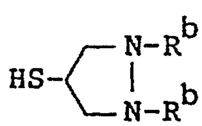

(III)

worin $R^b$ eine Amino-Schutzgruppe bedeutet, unter Bildung einer Verbindung der folgenden Formel

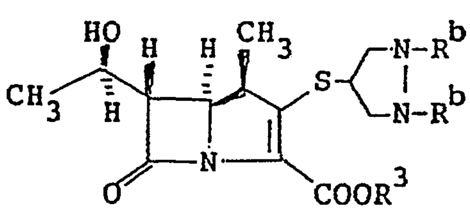

(IV)

worin $R^3$ und $R^b$ die oben angegebenen Bedeutungen besitzen, umgesetzt wird und aus der Verbindung der Formel (IV) die Schutzgruppen $R^3$ und $R^b$ entfernt werden.

10. Verfahren zur Herstellung von (1R,5S,6S)-2-[(6, 7-Dihydro-5H-pyrazolo[1, 2-a][1,2,4]triazolium-6-yl)]thio-6[(R)-1-hydroxyethyl]-1-methyl-carbapenem-3-carboxylat, dargestellt durch die folgende Formel

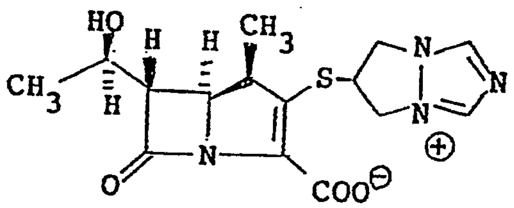

(I-2)

oder eines pharmakologisch annehmbaren Salzes davon; dadurch gekennzeichnet , daß eine Verbindung der folgenden Formel

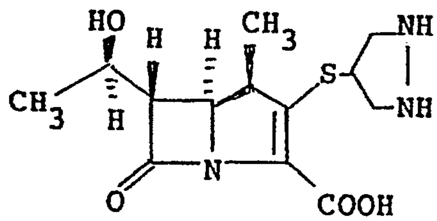

(I-1)

mit Ameisenimidinsäureester umgesetzt wird.

11. Medikament, dadurch gekennzeichnet , daß es (1R,5S,6S)-2-[(6, 7-Dihydro-5H-pyrazolo [1, 2a][1,2,4]triazolium-6-yl)]thio-6-[(R)-1-hydroxyethyl]-1-methyl-carbapenem-3-carboxylat oder ein pharmakologisch annehmbares Salz davon enthält.

12. Antibakterielles Mittel, dadurch gekennzeichnet , daß es (1R,5S,6S)-2-[(6, 7-Dihydro-5-H-pyrazolo[1,2-a][1,2,4]triazolium-6-yl)]thio-6-[(R)-1-hydroxyethyl]-1-methyl-carbapenem-3-carboxylat oder ein pharmakologisch annehmbares Salz davon enthält.

13. Die Verwendung der Verbindung nach einem der Ansprüche 3 oder 4 zur Herstellung antibakterieller Arzneimittel.

**Patentansprüche für den benannten Vertragsstaat: ES**

1. Verfahren zur Herstellung von (1R,5S,6S)-2-[4-pyrazolidinyl]-thio-6-[(R)-1-hydroxyethyl]-1-methyl-carbapenem-3-carbonsäure, dargestellt durch die folgende Formel

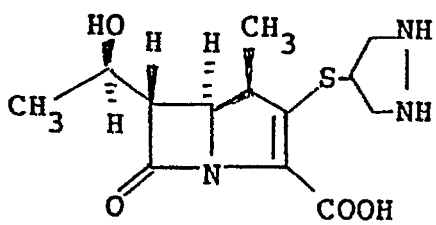

(I-1)

oder eines pharmakologisch annehmbaren Salzes davon, dadurch gekennzeichnet , daß eine Verbindung der folgenden Formel

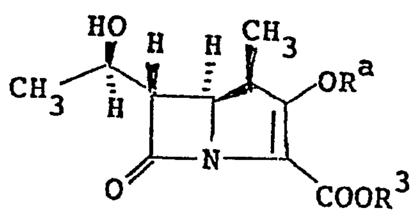

(II)

worin $R^3$ eine Carboxyl-Schutzgruppe und $R^a$ eine Acylgruppe bedeuten, mit einem Mercaptoreagens,

dargestellt durch die folgende Formel

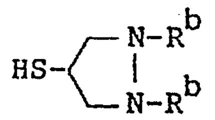

(III)

worin $R^b$ eine Amino-Schutzgruppe bedeutet, unter Bildung einer Verbindung der folgenden Formel

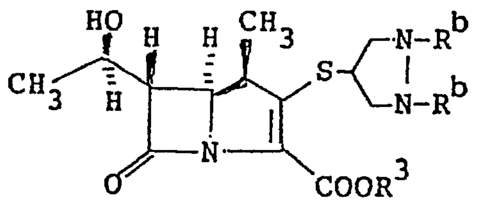

(IV)

worin $R^3$ und $R^b$ die oben angegebenen Bedeutungen besitzen, umgesetzt wird und aus der Verbindung der Formel (IV) die Schutzgruppen $R^3$ und $R^b$ entfernt werden.

2. Verfahren zur Herstellung von (1R,5S,6S)-2-[(6, 7-Dihydro-5H-pyrazolo[1, 2-a][1,2,4]triazolium-6-yl)]thio-6-[(R)-1-hydroxyethyl]-1-methyl-carbapenem-3-carboxylat, dargestellt durch die folgende Formel

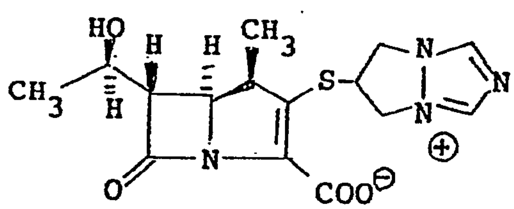

(I-2)

oder eines pharmakologisch annehmbaren Salzes davon; dadurch gekennzeichnet , daß eine Verbindung der folgenden Formel

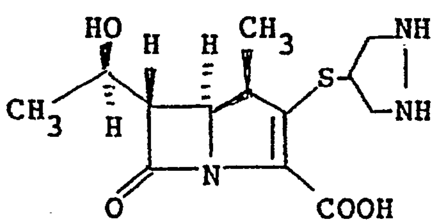

(I-1)

mit Ameisenimidinsäureester umgesetzt wird.

3. Verfahren nach Anspruch 1, dadurch gekennzeichnet , daß als Produkt (1R,5S,6S)-2-[4-pyrazolidinyl]-thio-6-[(R)-1-hydroxyethyl]-1-methyl-carbapenem-3-carbonsäure der folgenden Formel

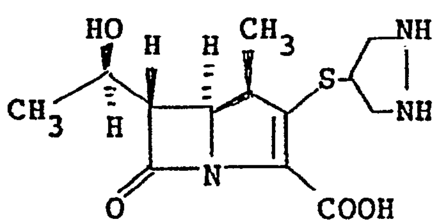

(I-1)

oder ein pharmakologisch annehmbares Salz davon hergestellt wird.

4. Verfahren nach Anspruch 2, dadurch gekennzeichnet , daß als Produkt (1R,5S,6S)-2-[(6, 7-Dihydro-5H-pyrazolo[1, 2-a][1,2,4]-triazolium-6-yl)]thio-6[(R)-1-hydroxyethyl]-1-methyl-carbapenem-3-carboxylat der folgenden Formel

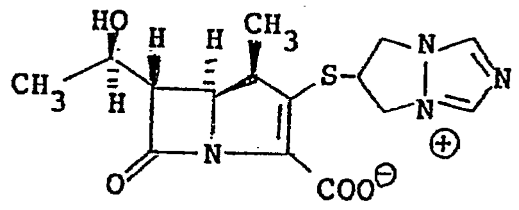

(I-2)

oder ein pharmakologisch annehmbares Salz davon hergestellt wird.

5. Verfahren nach Anspruch 4, dadurch gekennzeichnet , daß die Verbindung der Formel (1-2) in kristalliner Form oder ein pharmakologisch annehmbares Salz davon hergestellt wird.

6. Verfahren nach Anspruch 2, dadurch gekennzeichnet , daß das Produkt (1R,5S,6S)-2-[(6, 7-Dihydro-5H-pyrazolo[1, 2-a][1,2,4]triazolium-6-yl)]thio-6[(R)-1-hydroxyethyl]-1-methyl-carbapenem-3-carboxylat oder ein pharmakologisch annehmbares Salz davon als pharmazeutisch aktive Substanz hergestellt wird.

7. Verfahren nach Anspruch 2, dadurch gekennzeichnet , daß das Produkt (1R,5S,6S)-2-[(6, 7-Dihydro-5H-pyrazolo[1, 2-a][1,2,4]triazolium-6-yl)]thio-6[(R)-1-hydroxyethyl]-1-methyl-carbapenem-3-carboxylat oder ein pharmakologisch annehmbares Salz davon als antibakterielles Mittel hergestellt wird.

8. Verfahren zur Herstellung eines Arzneimittels, dadurch gekennzeichnet , daß (1R,5S,6S)-2-[(6, 7-Dihydro-5H-pyrazolo[1, 2-a][1,2,4]triazolium-6-yl)]thio-6[(R)-1-hydroxyethyl]-1-methyl-carbapenem-3-carboxylat oder ein pharmakologisch annehmbares Salz davon und ein pharmakologisch annehmbarer Träger und/oder ein Verdünnungsmittel vermischt werden.

9. Verfahren zur Herstellung eines antibakteriellen Mittels, dadurch gekennzeichnet , daß (1R,5S,6S)-2-[(6, 7-Dihydro-5H-pyrazolo[1, 2-a][1,2,4]triazolium-6-yl)]-thio-6-[(R)-1-hydroxyethyl]-1-methyl-carbapenem-3-carboxylat oder ein pharmakologisch annehmbares Salz davon und ein pharmakologisch annehmbarer Träger und/oder ein Verdünnungsmittel vermischt werden.

**Patentansprüche für den benannten Vertragsstaat: GR**

1. Ein (1R,5S,6S)-2-substituiertes Thio-6-[(R)-1-hydroxyethyl]-1-methyl-carbapenem-3-carbonsäurederivat, dargestellt durch die folgende Formel

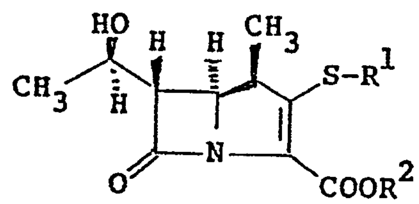

(I)

worin $R^1$ eine Gruppe der Formel

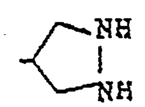

ou

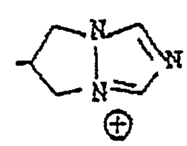

bedeutet und $R^2$ ein Wasserstoffatom oder eine anionische Ladung bedeutet, oder ein Salz davon.

2. Verbindung nach Anspruch 1, nämlich (1R,5S,6S)-2-[4-pyrazolidinyl]-thio-6-[(R)-1-hydroxyethyl]-1-methyl-carbapenem-3-carbonsäure der folgenden Formel

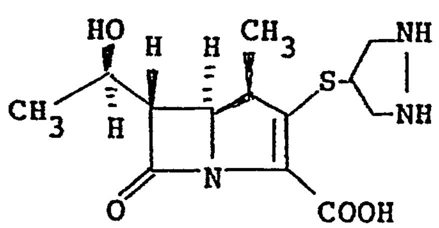

(I-1)

oder ein Salz davon.

3. Verbindung nach Anspruch 1, nämlich (1R,5S,6S)-2[(6, 7-Dihydro-5H-pyrazolo[1, 2-a][1,2,4]-tria-

zolium-6-yl)]thio-6-[(R)-1-hydroxyethyl]-1-methyl-carbapenem-3-carboxylat der folgenden Formel

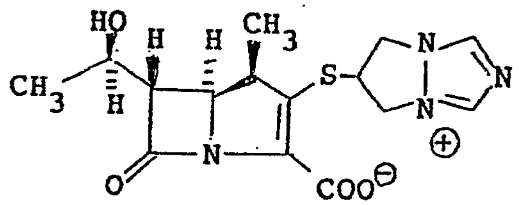
(I-2)

oder ein Salz davon.

4. Verbindung nach Anspruch 3, nämlich die Verbindung der Formel (1-2) in kristalliner Form, oder ein Salz davon.

5. Verbindung nach irgendeinem der Ansprüche 3 oder 4 als antibakterielles Mittel.

6. Verwendung von (1R,5S,6S)-2-[(6, 7-Dihydro-5H-pyrazolo[1, 2-a][1,2,4]triazolium-6-yl)]thio-6-[(R)-1-hydroxyethyl]-1-methyl-carbapenem-3-carboxylat oder ein pharmakologisch annehmbares Salz davon als antibakterielles Mittel.

7. Verfahren zur Herstellung von (1R,5S,6S)-2-[4-Pyrazolidinyl]-thio-6-[(R)-1-hydroxyethyl]-1-methyl-carbapenem-3-carbonsäure der folgenden Formel

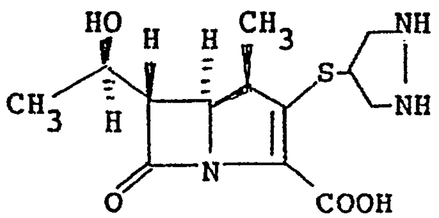
(I-1)

oder eines pharmakologisch annehmbaren Salzes davon; dadurch gekennzeichnet , daß eine Verbindung der folgenden Formel

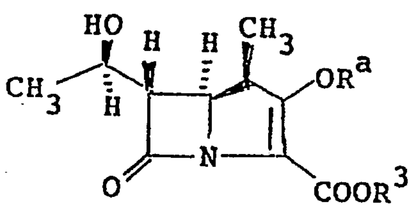
(II)

worin $R^3$ eine Carboxyl-Schutzgruppe und $R^a$ eine Acylgruppe bedeuten, mit einem Mercaptoreagens, dargestellt durch die folgende Formel

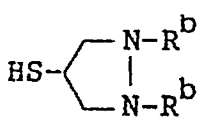
(III)

worin $R^b$ eine Amino-Schutzgruppe bedeutet, unter Bildung einer Verbindung der folgenden Formel

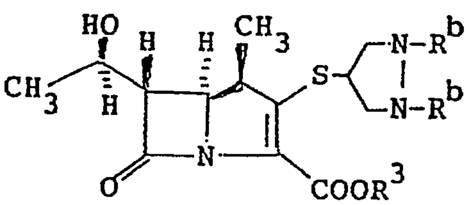
(IV)

worin $R^3$ und $R^b$ die oben angegebenen Bedeutungen besitzen, umgesetzt wird und aus der Verbindung der Formel (IV) die Schutzgruppen $R^3$ und $R^b$ entfernt werden.

8. Verfahren zur Herstellung von (1R,5S,6S)-2-[(6, 7-Dihydro-5H-pyrazolo[1, 2-a][1,2,4]triazolium-6-yl)]thio-6[(R)-1-hydroxyethyl]-1-methyl-carbapenem-3-carboxylat der folgenden Formel

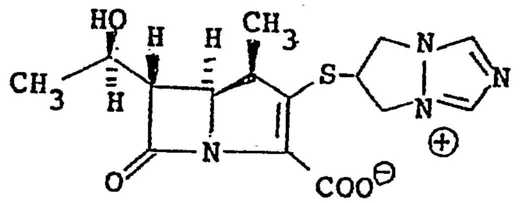

(I-2)

oder eines pharmakologisch annehmbaren Salzes davon; dadurch gekennzeichnet , daß eine Verbindung der folgenden Formel

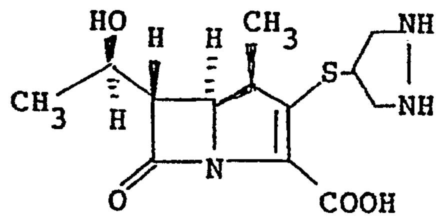

(I-1)

mit Ameisenimidinsäureester umgesetzt wird.

9. Verfahren zur Herstellung eines Gemisches, dadurch gekennzeichnet , daß (1R,5S,6S)-2-[(6, 7-Dihydro-5H-pyrazolo[1, 2-a][1,2,4]triazolium-6-yl)]thio-6[(R)-1-hydroxyethyl]-1-methyl-carbapenem-3-carboxylat oder ein pharmakologisch annehmbares Salz davon und ein pharmakologisch annehmbarer Träger und/oder ein Verdünnungsmittel vermischt werden.

10. Verfahren zur Herstellung eines antibakteriellen Mittels, dadurch gekennzeichnet , daß (1R,5S,6S)-2-[(6, 7-Dihydro-5H-pyrazolo[1, 2-a][1,2,4]triazolium-6-yl)]thio-6-[(R)-1-hydroxyethyl]-1-methyl-carbapenem-3-carboxylat oder ein pharmakologisch annehmbares Salz davon und ein pharmakologisch annehmbarer Träger und/oder ein Verdünnungsmittel vermischt werden.

11. Verwendung einer Verbindung nach einem der Ansprüche 3 oder 4 zur Herstellung antibakterieller Mittel.

**Revendications pour les Etats contractants: AT, BE, CH, DE, FR, GB, IT, LI, LU, NL, SE**

1. Dérivé d'acide (1R,5S,6S)-6-[(R)-1-hydroxy-éthyl]-1-méthyl-carbapénème-3-carboxylique substitué en position 2 par un groupe thio substitué, représenté par la formule suivante

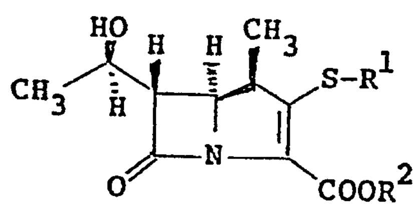

(I)

dans laquelle $R^1$ représente un radical de formule

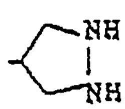

ou

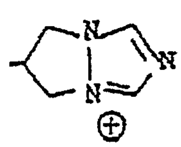

et $R^2$ est un atome d'hydrogène ou une charge anionique, ou un sel pharmacologiquement acceptable de celui-ci.

2. Composé de la revendication 1, qui est l'acide (1R,5S,6S)-2-[4-pyrazolidinyl]thio-6-[(R)-1-hydroxyéthyl]-1-méthyl-carbapénème-3-carboxylique de formule suivante

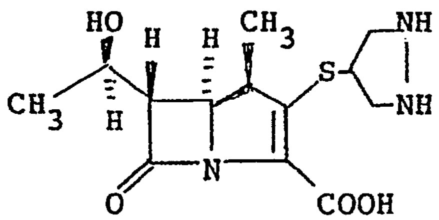

(I-1)

ou un sel pharmacologiquement acceptable de celui-ci.

3. Composé de la revendication 1, qui est le (1R,5S,6S)-2-[(6, 7-dihydro-5H-pyrazolo[1, 2-a][1,2,4]triazolium-6-yl)]thio-6-[(R)-1-hydroxyéthyl]-1-méthylcarbapénème-3-carboxylate de formule suivante

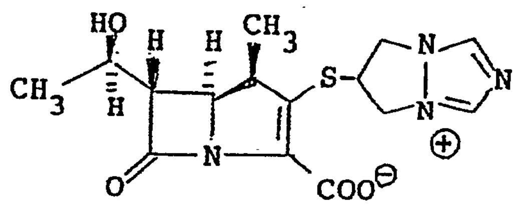

(I-2)

ou un sel pharmacologiquement acceptable.

4. Composé de la revendication 3, qui est le composé de formule (I-2) sous une forme cristalline, ou un sel pharmacologiquement acceptable.

5. Composé de l'une quelconque des revendications 3 et 4, en tant que substance pharmaceutiquement active.

6. (1R,5S,6S)-2-[(6, 7-dihydro-5H-pyrazolo[1, 2-a][1,2,4]triazolium-6-yl)]thio-6-[(R)-1-hydroxyéthyl]-1-méthyl-carbapénème-3-carboxylate ou un sel pharmacologiquement acceptable de celui-ci, en tant que substance pharmaceutiquement active.

7. Composé de l'une quelconque des revendications 3 et 4, en tant qu'agent antibactérien.

8. (1R,5S,6S)-2-[(6, 7-dihydro-5H-pyrazolo[1,2-a][1,2,4]triazolium-6-yl)]thio-6-[(R)-1-hydroxyéthyl]-1-méthyl-carbapénème-3-carboxylate ou un sel pharmacologiquement acceptable de celui-ci, en tant qu'agent antibactérien.

9. Procédé pour la production de l'acide (1R,5S,6S)-2-[4-pyrazolidinyl]thio-6-[(R)-1-hydroxyéthyl]-1-méthyl-carbapénème-3-carboxylique représenté par la formule suivante

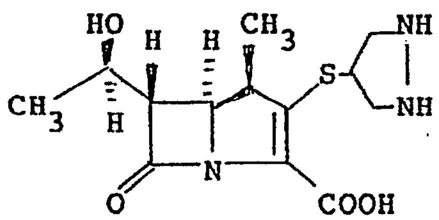

(I-1)

ou d'un sel pharmacologiquement acceptable de celui-ci; qui consiste à faire réagir un composé représenté par la formule suivante

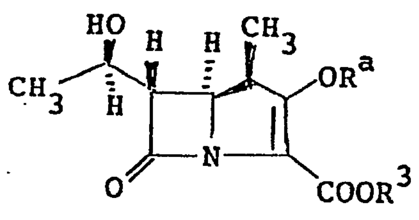

(II)

dans laquelle $R^3$ est un groupe protecteur de fonction carboxyle et $R^a$ est un groupe acyle, avec un réactif à groupe mercapto représenté par la formule suivante

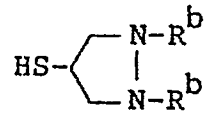

(III)

dans laquelle $R^b$ est un groupe protecteur de fonction amino, pour obtenir un composé représenté par la formule suivante

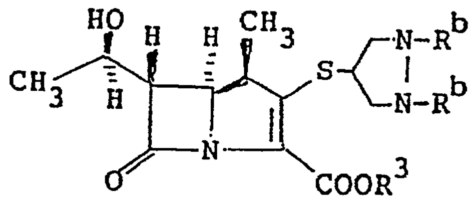

(IV)

dans laquelle $R^3$ et $R^b$ ont les mêmes significations que ci-dessus, et à soumettre le composé de formule (IV) à une élimination des groupes protecteurs $R^3$ et $R^b$.

10. Procédé pour la production du (1R,5S,6S)-2[(6, 7-dihydro-5H-pyrazolo[1, 2-a][1,2,4]triazolium-6-yl)]thio-6-[(R)-1-hydroxyéthyl]-1-méthyl-carbapénème-3-carboxylate représenté par la formule suivante

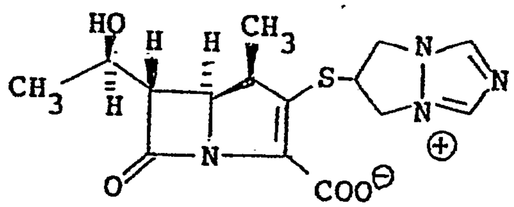

(I-2)

ou d'un sel pharmacologiquement acceptable de celui-ci ; qui consiste à faire réagir un composé représenté par la formule suivante

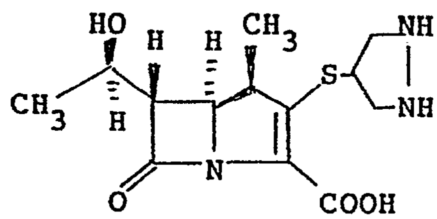

(I-1)

avec un ester d'acide formimidique.

11. Médicament comprenant du (1R,5S,6S)-2-[(6, 7-dihydro-5H-pyrazolo[1, 2-a][1,2,4]triazolium-6-yl)]thio-6-[(R)-1-hydroxyéthyl]-1-méthyl-carbapénème-3-carboxylate ou un sel pharmacologiquement acceptable de celui-ci.

12. Agent antibactérien comprenant du (1R,5S,6S)2-[(6, 7-dihydro-5H-pyrazolo[1, 2-a][1,2,4]triazolium-6-yl]thio-6-[(R)-1-hydroxyéthyl]-1-méthyl-carbapénème-3-carboxylate ou un sel pharmacologiquement acceptable de celui-ci.

13. Utilisation du composé selon l'une quelconque des revendications 3 et 4 pour la fabrication de médicaments antibactériens.

**Revendications pour l'Etat contractant: ES**

1. Procédé pour la production de l'acide (1R,5S,6S)-2-[4-pyrazolidinyl]thio-6-[(R)-1-hydroxyéthyl]-1-méthyl-carbapénème-3-carboxylique représenté par la formule suivante

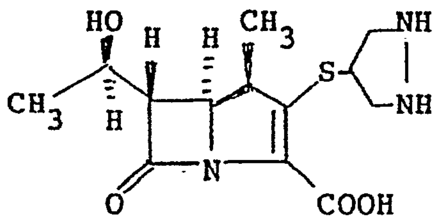

(I-1)

ou d'un sel pharmacologiquement acceptable de celui-ci , qui consiste à faire réagir un composé représenté par la formule suivante

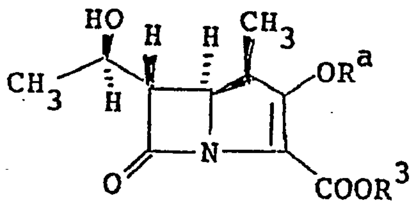

(II)

dans laquelle $R^3$ est un groupe protecteur de fonction carboxyle et $R^a$ est un groupe acyle, avec un réactif à groupe mercapto représenté par la formule suivante

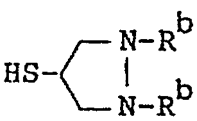

(III)

dans laquelle $R^b$ est un groupe protecteur de fonction amino, pour obtenir un composé représenté par la formule suivante

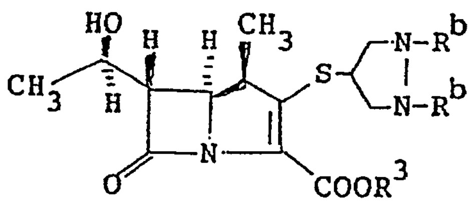

(IV)

dans laquelle $R^3$ et $R^b$ ont les mêmes significations que ci-dessus, et à soumettre le composé de formule (IV) à une élimination des groupes protecteurs $R^3$ et $R^b$.

2. Procédé pour la production du (1R,5S,6S)-2[(6, 7-dihydro-5H-pyrazolo[1, 2-a][1,2,4]triazolium-6-yl)]thio-6-[(R)-1-hydroxyéthyl]-1-méthyl-carbapénème-3-carboxylate représenté par la formule suivante

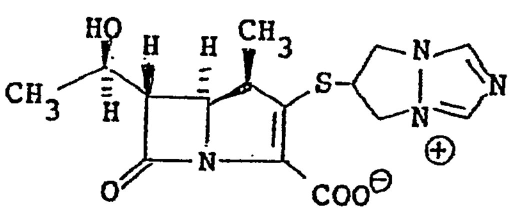

(I-2)

ou d'un sel pharmacologiquement acceptable de celui-ci, qui consiste à faire réagir un composé représenté par la formule suivante

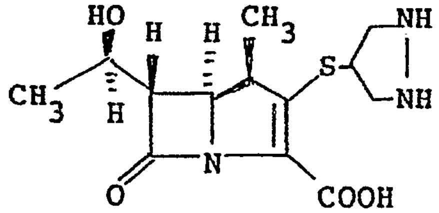

(I-1)

avec un ester d'acide formimidique.

3. Procédé de la revendication 1, dans lequel on obtient comme produit l'acide (1R,5S,6S)-2-[4-pyrazolidinyl]thio-6-[(R)-1-hydroxyéthyl]-1-méthyl-carbapénème-3-carboxylique de formule suivante

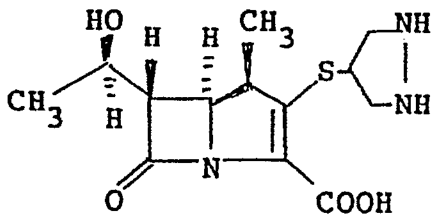

(I-1)

ou un sel pharmacologiquement acceptable de celui-ci.

4. Procédé de la revendication 2, dans lequel on obtient comme produit le (1R,5S,6S)-2-[(6, 7-dihydro-5H-pyrazolo(1, 2-a][1,2,4]triazolium-6-yl)]thio-6-[(R)-1-hydroxyéthyl]-1-méthyl-carbapénème-3-carboxylate de formule suivante

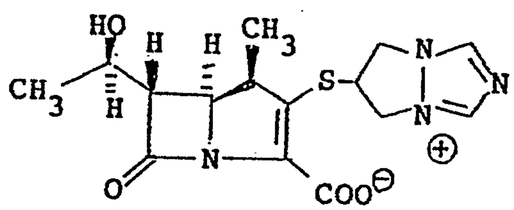

(I-2)

ou un sel pharmacologiquement acceptable de celui-ci.

5. Procédé de la revendication 4, dans lequel on obtient le composé de formule (I-2) sous une forme cristalline, ou un sel pharmacologiquement acceptable.

6. Procédé selon la revendication 2, dans lequel on obtient comme produit le (1R,5S,6S)-2-[(6, 7-dihydro-5H-pyrazolo[1, 2-a][1,2,4]triazolium-6-yl)]thio-6-[(R)-1-hyodroxyéthyl]-1-méthyl-carbapénème-3-carboxylate ou un sel pharmacologiquement acceptable de celui-ci, en tant que substance pharmaceutiquement active.

7. Procédé selon la revendication 2, dans lequel on obtient comme produit le (1R,5S,6S)-2-[(6, 7-dihydro-5H-pyrazolo[1, 2-a][1,2,4]triazolium-6-yl)]thio-6-[(R)-1-hydroxyéthyl]-1-méthyl-carbapénème-3-carboxylate ou un sel pharmacologiquement acceptable de celui-ci, en tant qu'agent antibactérien.

8. Procédé pour la préparation d'un médicament, consistant à mélanger du (1R,5S,6S)-2-[(6, 7-dihydro-5H-pyrazolo[1, 2-a][1,2,4]triazolium-6-yl)]thio-6-[(R)-1-hydroxyéthyl]-1-méthyl-carbapénème-3-carboxylate ou un sel pharmacologiquement acceptable de celui-ci et un support et/ou un diluant pharmacologiquement acceptables.

9. Procédé pour la préparation d'un agent antibactérien, consistant à mélanger du (1R,5S,6S)-2-[(6, 7-dihydro-5H-pyrazolo[1, 2-a][1,2,4]triazolium-6-yl)]thio-6[(R)-1-hydroxyéthyl]-1-méthyl-carbapénème-3-carboxylate un sel pharmacologiquement acceptable de celui-ci et un support et/ou un diluant pharmacologiquement acceptables.

**Revendications pour l'Etat contractant: GR**

1. Dérivé d'acide (1R,5S,6S)-6-[(R)-1-hydroxyéthyl]-1-méthyl-carbapénème-3-carboxylique substitué en position 2 par un groupe thio substitué, représenté par la formule suivante

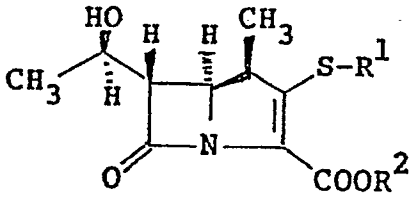

(I)

dans laquelle $R^1$ représente un radical de formule

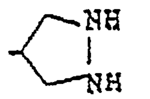

ou

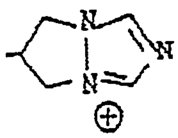

et $R^2$ est un atome d'hydrogène ou une charge anionique, ou un sel de celui-ci.

2. Composé de la revendication 1, qui est l'acide (1R,5S,6S)-2-[4-pyrazolidinyl]thio-6-[(R)-1-hydroxyéthyl]-1-méthyl-carbapénème-3-carboxylique de formule suivante

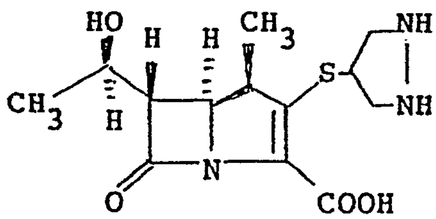

(I-1)

ou un sel de celui-ci.

3. Composé de la revendication 1, qui est le (1R,5S,6S)-2-[(6, 7-dihydro-5H-pyrazolo[1, 2-a][1,2,4]triazolium-6-yl)]thio-6-[(R)-1-hydroxyéthyl]-1-méthyl-carbapénème-3-carboxylate de formule suivante

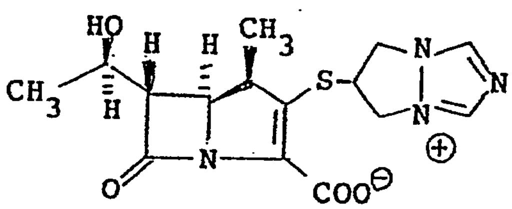

(I-2)

ou un sel de celui-ci.

4. Composé de la revendication 3, qui est le composé de formule (I-2) sous une forme cristalline, ou un sel de celui-ci.

5. Composé de l'une quelconque des revendications 3 et 4, en tant qu'agent antibactérien.

6. Utilisation du (1R,5S,6S)-2-[(6, 7-dihydro-5H-pyrazolo[1, 2-a][1,2,4]triazolium-6-yl)]thio-6-[(R)-1-hydroxyéthyl]-1-méthyl-carbapénème-3-carboxylate ou d'un sel pharmacologiquement acceptable de celui-ci, en tant qu'agent antibactérien.

7. Procédé pour la production de l'acide (1R,5S,6S)-2-[4-pyrazolidinyl]thio-6-[(R)-1-hydroxyéthyl]-1-méthyl-carbapénème-3-carboxylique représenté par la formule suivante

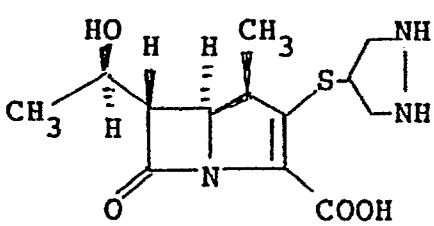

(I-1)

ou d'un sel pharmacologiquement acceptable de celui-ci; qui consiste à faire réagir un composé représenté par la formule suivante

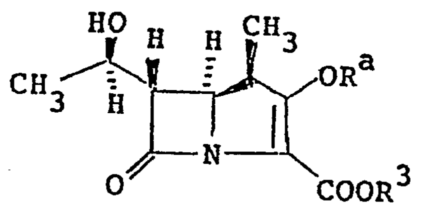

(II)

dans laquelle $R^3$ est un groupe protecteur de fonction carboxyle et $R^a$ est un groupe acyle, avec un réactif à groupe mercapto représenté par la formule suivante

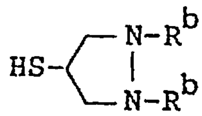

(III)

dans laquelle $R^b$ est un groupe protecteur de fonction amino, pour obtenir un composé représenté par la formule suivante

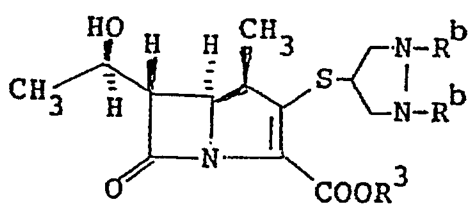

(IV)

dans laquelle $R^3$ et $R^b$ ont les mêmes significations que ci-dessus, et à soumettre le composé de formule (IV) à une élimination des groupes protecteurs $R^3$ et $R^b$.

8. Procédé pour la production du (1R,5S,6S)-2[(6, 7-dihydro-5H-pyrazolo[1, 2-a][1,2,4]triazolium-6-yl)]thio-6-[(R)-1-hydroxyéthyl]-1-méthyl-carbapénème-3-carboxylate représenté par la formule suivante

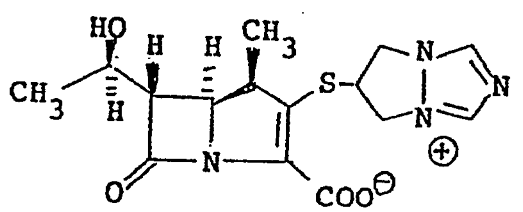

(I-2)

ou d'un sel pharmacologiquement acceptable de celui-ci, qui consiste à faire réagir un composé représenté par la formule suivante

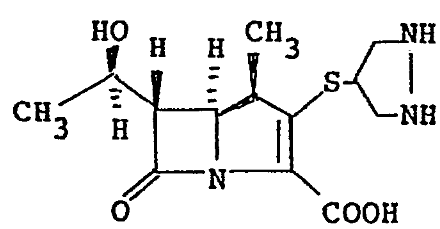

(I-1)

avec un ester d'acide formimidique.

9. Procédé pour la préparation d'un mélange, consistant à mélanger du (1R,5S,6S)-2-[(6, 7-dihydro-5H-pyrazolo[1, 2-a][1,2,4]triazolium-6-yl)]thio-6-[ (R)-1-hydroxyéthyl]-1-méthyl-carbapénème-3-carboxylate ou un sel pharmacologiquement acceptable de celui-ci et un support et/ou un diluant pharmacologiquement acceptables.

10. Procédé pour la préparation d'un agent antibactérien, consistant à mélanger du (1R,5S,6S)-2-[(6, 7-dihydro-5H-pyrazolo[1, 2-a][1,2,4]triazolium-6-yl)]thio-6-[(R)-1-hydroxyéthyl]-1-méthyl-carbapénème-3-carboxylate ou un sel pharmacologiquement acceptable de celui-ci et un support et/ou un diluant pharmacologiquement acceptables.

11. Utilisation d'un composé selon l'une quelconque des revendications 3 et 4 pour la fabrication d'agents antibactériens.